(19) Europäisches Patentamt
European Patent Office
Office européen des brevets

(11) **EP 2 005 158 B1**

(12) **EUROPEAN PATENT SPECIFICATION**

(45) Date of publication and mention
of the grant of the patent:
**15.12.2010 Bulletin 2010/50**

(51) Int Cl.:
***G01N 33/49*** *(2006.01)*

(21) Application number: **07723794.9**

(22) Date of filing: **30.03.2007**

(86) International application number:
**PCT/EP2007/002852**

(87) International publication number:
**WO 2007/112961 (11.10.2007 Gazette 2007/41)**

(54) **DISPENSER FOR FLATTENED ARTICLES**

SPENDER FÜR FLACHE ARTIKEL

DISTRIBUTEUR D'ARTICLES APLATIS

(84) Designated Contracting States:
**AT BE BG CH CY CZ DE DK EE ES FI FR GB GR
HU IE IS IT LI LT LU LV MC MT NL PL PT RO SE
SI SK TR**

(30) Priority: **31.03.2006 US 394776**

(43) Date of publication of application:
**24.12.2008 Bulletin 2008/52**

(73) Proprietors:
• **Roche Diagnostics GmbH
68305 Mannheim (DE)**
Designated Contracting States:
**DE**
• **F.Hoffmann-La Roche AG
4070 Basel (CH)**

(72) Inventors:
• **FUNKE, Tom
Carmel, IN 46032 (US)**
• **JOSEPH, Abner, David
Carmel, IN 46033 (US)**
• **ARNOLD, Peter, L.
64646 Heppenheim (DE)**
• **YODER, Randy Edward
Summerville, SC 29485 (US)**
• **STRANKO, Scott, K.
Indianapolis, IN 46241 (US)**

(56) References cited:
**WO-A-03/024514            WO-A1-2006/009534
US-A1- 2002 104 849      US-A1- 2005 281 706**

## Description

### FIELD OF THE INVENTION

The present invention relates generally to dispensers and more particularly to dispensers for flattened articles such as test strips.

### BACKGROUND

Test strips or biosensors for measuring the presence or concentrations of selected analytes in test samples are well known. Typically, several of the test strips are packaged and stored in a disposable vial having a lid that snaps off or unscrews to open. Desiccant material is normally packaged within the vial to maintain the test strips dry. To conduct a test, the user must open the vial and remove a test strip. The strip is then typically inserted into a meter and a fluid sample (normally whole blood) is deposited onto it. The meter then measures the concentration of analyte using photometric or electrochemical methods. When the test is finished, the strip is removed from the meter and discarded. Also, before beginning use of a new vial of test strips, a user is typically given the opportunity to code the meter for the particular test strips, in other words input identifying information about the test strips (such as a calibration date, lot number, expiration date, etc.) into the meter. This coding is sometimes accomplished via a memory chip provided with the test strips that the user then manually inserts into the meter.

Test strips are commonly used by diabetics to measure the level of glucose in their blood, which for most diabetics needs to be done three to four times per day, sometimes more frequently. Unfortunately, many diabetics develop complications from having the disease, such as impaired vision, loss of hand-eye coordination, and loss of sensitivity and dexterity of the fingers and toes. These complications of the disease can make opening a test strip vial, extracting a single test strip, manipulating the test strip and/or coding the meter quite difficult.

Equally undesirably, opening a conventional vial of test strips exposes the strips to moisture in the atmosphere and causes the reagents contained in them to degrade much more quickly than if the vial remained sealed. This exposure significantly reduces shelf life.

Accordingly, there are needs for advances in this area of technology. More specifically, but not exclusively, there are needs for dispensers that ease handling of the test strips and/or that provide for automatic coding of the meter.

Dispensers providing solutions that ease handling of test strips including dispensers in accordance with the preamble of claim 1 are known from US 2002/104849 A1, WO 2006/009534 A1 and US 2005/281706 A1.

## SUMMARY

The present invention provides substantially moisture-proof, airtight dispensers for both storing and dispensing several diagnostic test strips being inter alia suitable for further means for automatically, coding a meter with test strip information. The invention is discussed more fully below.

## BRIEF DESCRIPTION OF THE DRAWINGS

The above-mentioned and other advantages of the present invention, and the manner of obtaining them, will become more apparent and the invention itself will be better understood by reference to the following description taken in conjunction with the accompanying drawings, wherein:

Figs. 1 and 2 are perspective views of an article dispenser in accordance with the prior art, illustrating the home and dispensed positions of the dispenser;

Figs. 1a and 2a are perspective views of an alternate example of an article dispenser in accordance with the prior art, illustrating the home and dispensed positions of the dispenser;

Fig. 3 is a perspective view in partial cross section of the dispenser shown in Figs. 1 and 2, illustrating the interior components of the dispenser;

Fig. 4 is an exploded perspective view of the dispenser shown in Figs. 1 and 2;

Figs. 5a and 5b are an enlarged fragmentary perspective view and a sectional view, respectively, of a seal in accordance with the prior art;

Fig. 6 is an exploded perspective view of a dispenser body or cassette in accordance with the prior art;

Figs. 7a-7d are side sectional views that illustrate the movement of a prior art dispenser from the home position to the dispense position and then back;

Fig. 8a is an exploded perspective view with portions broken away of the dispenser of Figs. 1 and 2;

Fig. 8b is an enlarged fragmentary view of a locking mechanism in accordance with the prior art;

Fig. 8c is an enlarged fragmentary perspective view illustrating the connection of a flexible arm member to a trigger;

Figs. 9a and 9b are enlarged fragmentary perspective views illustrating a lip seal cover ;

Figs. 10a and 10b are enlarged fragmentary perspective views in partial cross section illustrating a platform and a lip seal in accordance with the prior art;

Fig. 11a and 11b are enlarged fragmentary perspective views in partial cross section illustrating a lip seal and a reconfigured lip seal, respectively, in accordance with the prior art;

Figs. 12a and 12b are enlarged fragmentary perspective views in partial cross section illustrating another prior art lip seal and a reconfigured lip seal, respectively;

Fig. 13 is an enlarged fragmentary perspective view illustrating a cap in accordance with the prior art;

Fig. 14 is an enlarged fragmentary perspective view in partial cross section illustrating a cap and an exit in accordance with the prior art;

Figs. 15a-15c are enlarged fragmentary perspective views in partial cross section illustrating a flexible seal, a reconfigured flexible seal, and a reconfigured flexible seal with an article exiting the seal, respectively, in accordance with the prior art;

Fig. 16 is a side sectional view illustrating the movement of the prior art dispenser from the home position to an optional detent position; and

Figs. 17a-17c are perspective views illustrating a method of using the article dispenser with a meter that reads the articles that are dispensed.

Fig. 18 is a perspective view of an article dispenser with integrated RFID tag.

Figs. 19 and 20 are partial sectional views of an article dispenser of the flip top design in accordance with the present invention in the home and dispensed positions.

Fig. 21 is a partial cutaway assembly view of the outer housing and trigger assembly of the dispenser of Figs. 19 and 20.

Fig. 22 is a sectional view of the housing and trigger assembly of Fig. 21 being inserted over the cassette to form the dispenser of Figs. 19 and 20.

Fig. 23 is a partial cutaway view of the trigger of the dispenser of Figs. 19 and 20.

Fig. 24 is an enlarged partial perspective view of the pusher head portion of the dispenser of Figs. 19 and 20 received in one half of the inner cassette.

Fig. 25 is a view of the pusher head portion as shown in Fig. 24 with the outer housing shown in cutaway.

Fig. 26 is an enlarged view of the umbrella seal engaged with the outer housing to isolate the contents of the cassette from the external environment.

Corresponding reference characters indicate corresponding parts throughout the several views.

## DESCRIPTION OF THE PREFERRED EMBODIMENTS

For the purposes of prompting an understanding of the principles of the invention, reference will now be made to examples in accordance with the prior art and to specific embodiments illustrated herein and specific language will be used to describe the same. It will nevertheless be understood that no limitation of the scope of the invention is thereby intended, since the scope of the invention is defined by the claims. Any alterations and further modifications in the described processes or devices, and any further applications of the principles of the invention as described herein, are contemplated as would normally occur to one skilled in the art to which the invention relates.

In one form, the present invention provides devices for storing and individually dispensing test strips. As described more fully below, these test strip dispensers include a housing that contains a stack of test strips and has an exit through which the strips are dispensed. A trigger is coupled to an actuator for individually advancing test strips from the stack through the exit. Various arrangements of the trigger and actuator are contemplated. In certain illustrated embodiments, the actuator includes a pusher head that is slidably disposed above the test strip stack and, upon activation, frictionally engages the top strip in the stack to advance that strip out of the exit.

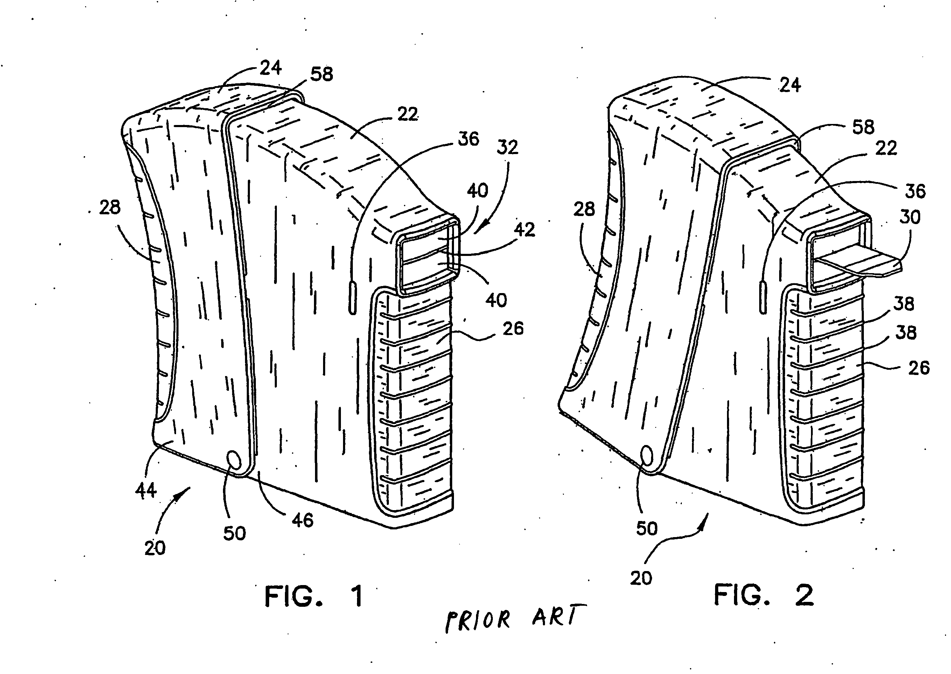

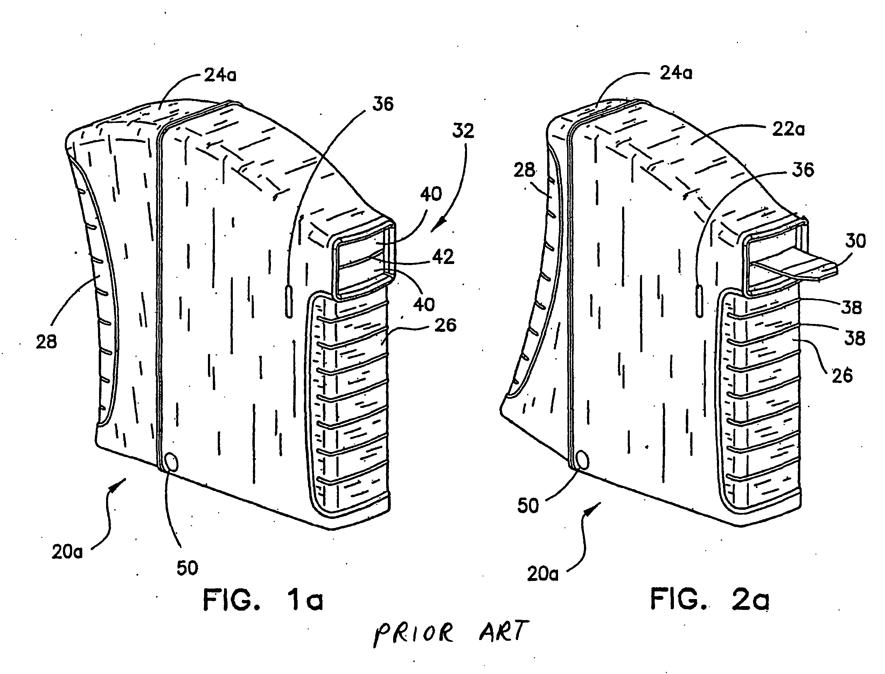

The trigger is adapted for ease of one-handed operation according to two basic arrangements. In the first arrangement in accordance with the prior art, the trigger is pivotally coupled to the housing and is activated by the user squeezing the trigger and housing together. In a preferred form of this arrangement, the trigger is located on one side of the dispenser with the test strips being ejected from the opposite side. An example of a dispenser according to this first arrangement is shown in, for example, Figs. 1 and 2.

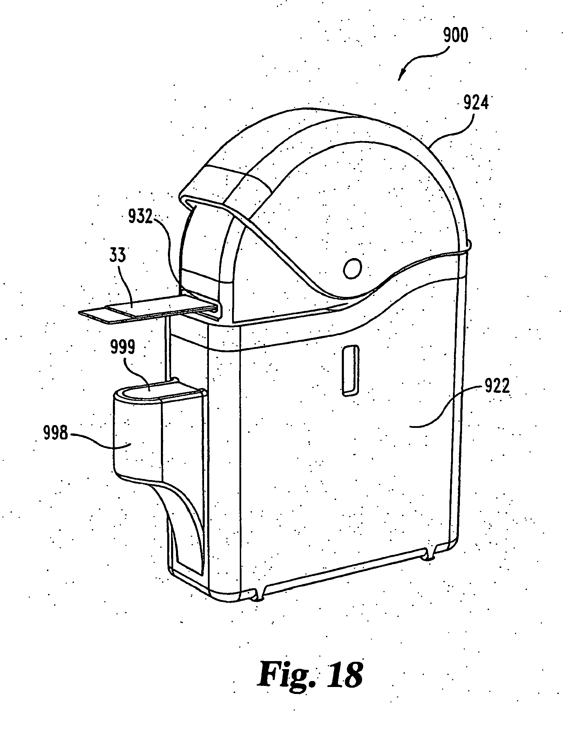

In the second arrangement representing an embodiment of the claimed invention, the trigger is pivotally coupled at the top of the housing and is configured to be activated by the user rotating the trigger relative to the housing, such as with the thumb of the user's hand. An example of a dispenser according to this second arrangement is shown in, for example, Fig. 18.

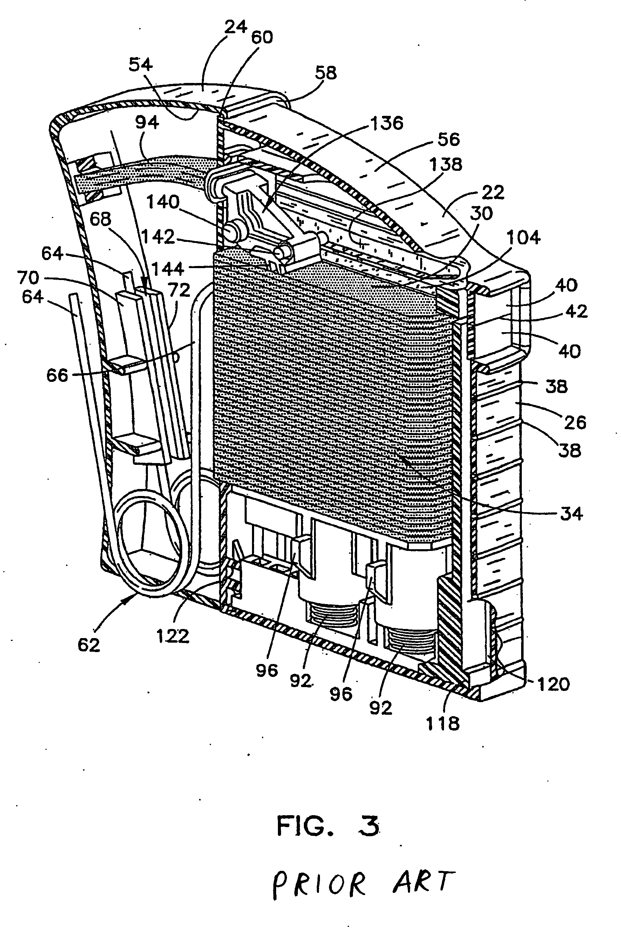

Turning now to Figs. 1 and 2, a specific example of a dispenser according to the first arrangement is illustrated. However, it is be understood that, unless otherwise indicated, the principles of construction and operation of this first arrangement can be applied in the second arrangement as would occur to those of skill in the art. Article dispenser 20 for dispensing flattened articles such as biosensors or test strips is shown having a main housing 22 pivotably connected to a trigger or rear housing 24. Fig. 1 illustrates a "home" position whereas Fig. 2 illustrates a "dispensed" position for dispenser 20. Dispenser 20 includes front grip section 26 and rear grip section 28, which, when pivoted together as shown in Fig. 2 and explained in detail below, causes an article 30 to be pushed at least partially out of exit 32. In the illustrated embodiment, article 30 is a test strip, e.g., an Accu-Chek® brand glucose test strip that is commercially available from the assignee of the present invention. However, it should be understood that the teachings of the dispenser disclosed herein may be employed for dispensers of other flattened articles. As shown in Fig. 3, main housing 22 carries a stack 34 of test strips 30 to be successively dispensed from dispenser 20, as explained below. Housing 22 includes a window 36 (Figs. 1 and 2) for viewing the quantity of articles 30 remaining in stack 34. Window 36 may be formed of any number of clear materials, e.g., clear polypropylene.

In the illustrated example, housing 22 and trigger 24 are formed of polypropylene and polystyrene, respectively, but it should be readily appreciated that many other plastics, composites or other materials may be used. Grip section 26 includes protruding ribs 38 that are preferably formed of a thermoplastic elastomer such as Santoprene®, available from Advanced Elastomer Systems, Akron, OH. Exit 32 includes flaps 40 that define a lip seal 42. Flaps 40 are also made from Santoprene® and are integrally formed with ribs 38 as illustrated in Fig. 3.

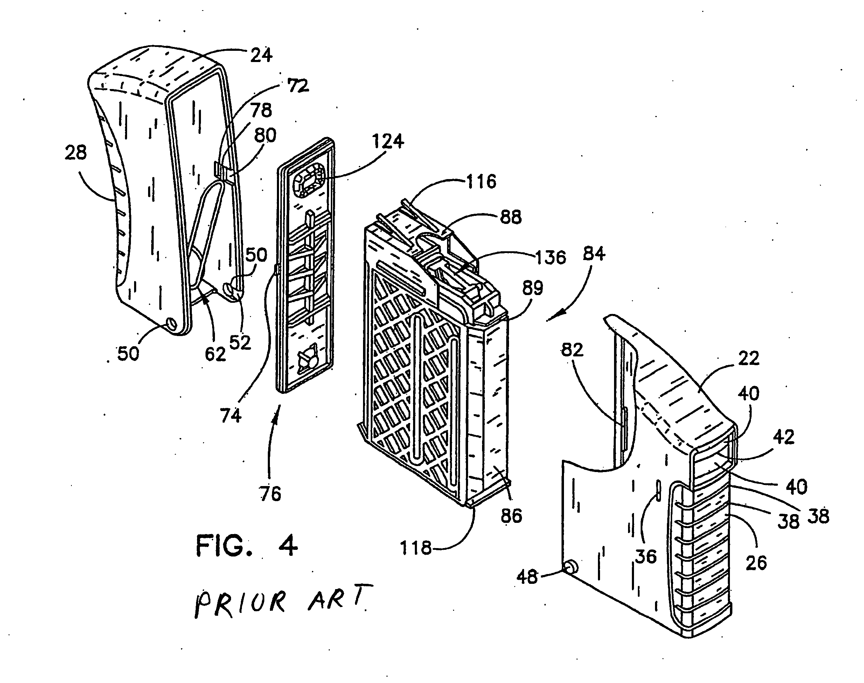

With further reference to Figs. 1 and 2, trigger 24 and housing 22 are pivotably connected at bottom portions 44 and 46, respectively, which results in housing 22 nesting within trigger 24 mainly at the top of dispenser 20. The bottom portions of the trigger and housing do not significantly nest together, which allows cassette 84 (Fig. 4) to be secured to the bottom of housing 22 without interference from trigger 24 when the two are pivoted together during dispensing. As shown in Fig. 4, housing 22 includes cylindrical posts 48 extending laterally therefrom that are rotatably received in corresponding cylindrical openings 50 in trigger 24. The inside surface of trigger 24 is formed with slots 52 that lead to openings 50, which aids assembly of dispenser 20 by allowing posts 48 to slide through slots 52. While the illustrated embodiment includes posts 48 formed on housing 22 and openings 50 on trigger 24, the situation could be reversed. Further, other means for making the pivotal connection between housing 22 and trigger 24, e.g., a hinge, could be substituted for the posts and openings.

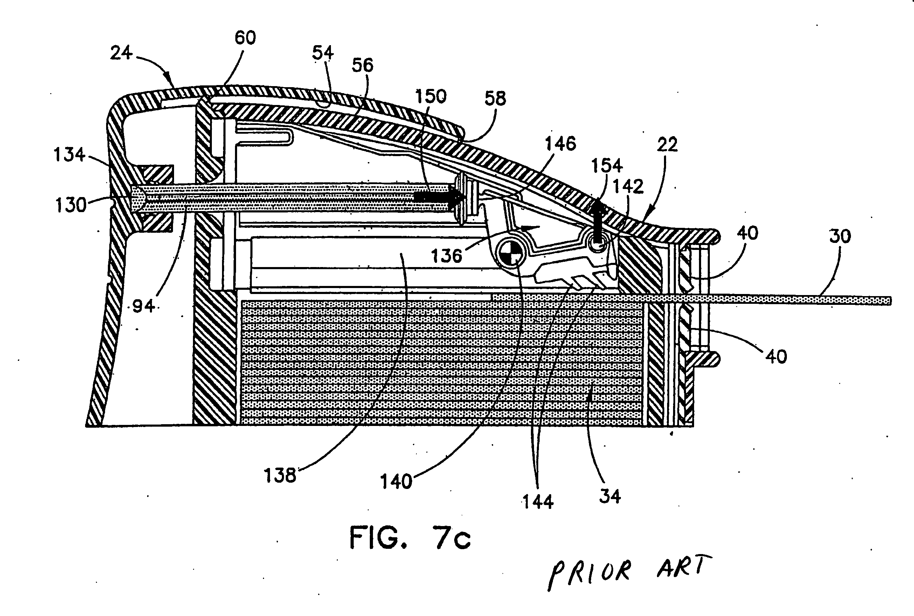

As shown in Figs. 1 and 2, since the pivotal connection is made at bottom portions 44 and 46, most of the movement of trigger 24 and housing 22 relative to one another occurs toward the top of dispenser 20. Trigger 24 has a profile that substantially matches that of housing 22 but is slightly larger, such that the inner surface of trigger 24 defines a receptacle for housing 22. As shown more clearly in Fig. 3, trigger 24 defines an arcuate inner wall 54 that has an arcuate profile similar to that of outer wall 56 defined by housing 22. Thus, when trigger 24 and housing 22 are pivoted toward one another, housing 22 partially nests within trigger 24 while an article 30 is expelled partially from dispenser 20 as shown in Fig. 2. As shown in Fig. 7c, inner wall 54 and outer wall 56 approximate concentric segments when trigger 24 and housing 22 are pivoted together.

While in the preferred example the trigger forms the receptacle, the pivoting housing principle could be employed if the situation were reversed. As shown in Figs. 1a and 2a, trigger 24a partially nests within a receptacle defined by main housing 22a as the two parts are pivoted together, and a test strip 30 is expelled partially from container 20a. Other variations of the housing would be recognized by one of ordinary skill in the art. As noted above, one advantage of this pivoting housing is that the actuable parts of the housing that cause a strip to be dispensed can be made as large as the dispenser itself. Thus, dispensing a strip with dispenser 20 or 20a requires only an overall squeeze of the hand, and which does not require individual movement of the fingers. As noted above, this is especially advantageous to diabetics who have lost finger sensation and dexterity and thus have trouble manipulating the small dials, caps and sliders present in other prior art dispensers.

As shown in Fig. 3, a torsion spring 62 has upstanding legs 64 and 66 which push against the interior of trigger 24 and housing 22, respectively, biasing the dispenser in the home position shown in Fig. 1. Spring 62 is held in place by means of channels 68 formed by spring retaining plates 70 and 72 formed in trigger 24 and which channels 68 captively hold legs 64. One of ordinary skill in the art would readily recognize many alternative spring mechanisms that could be configured and substituted for the torsion spring 62 of the illustrated example.

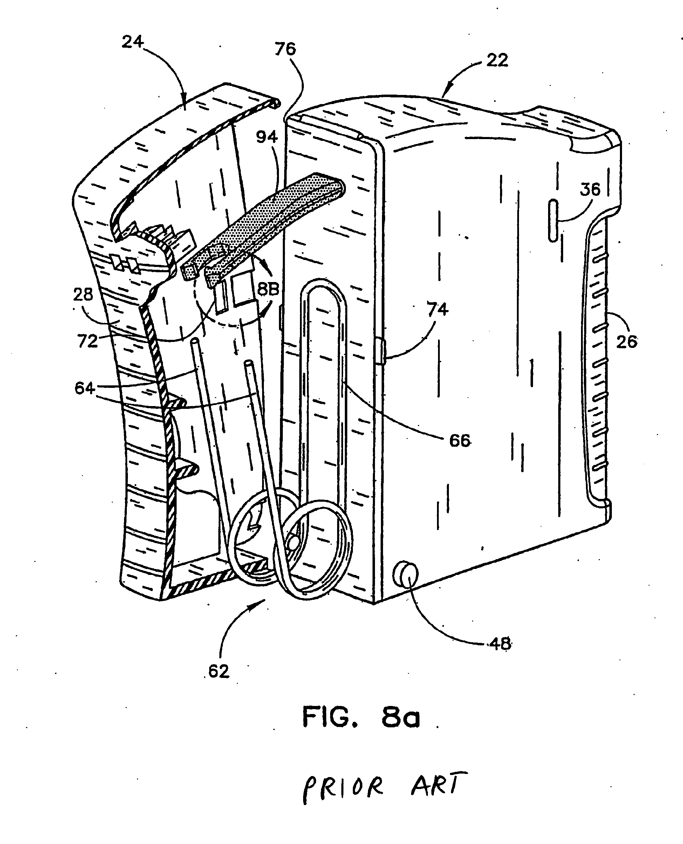

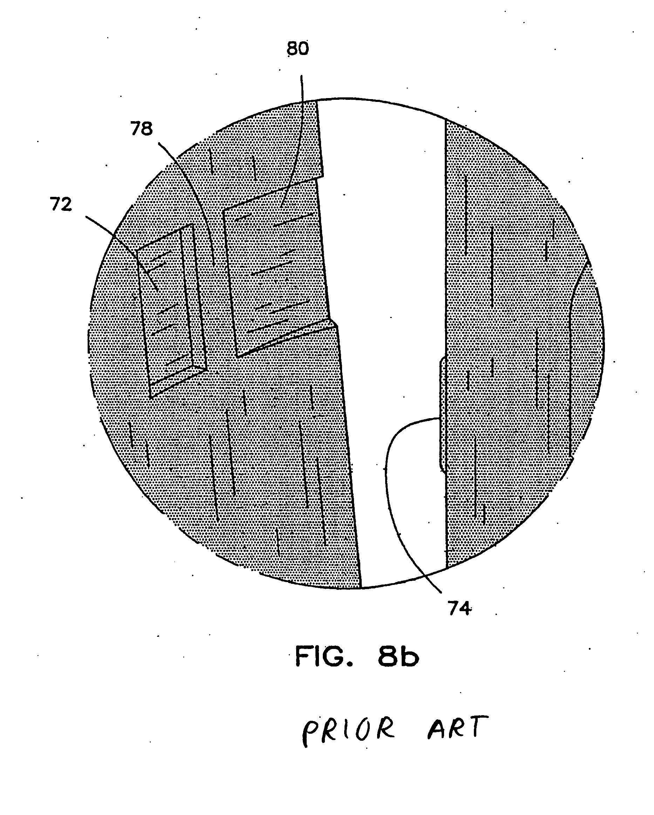

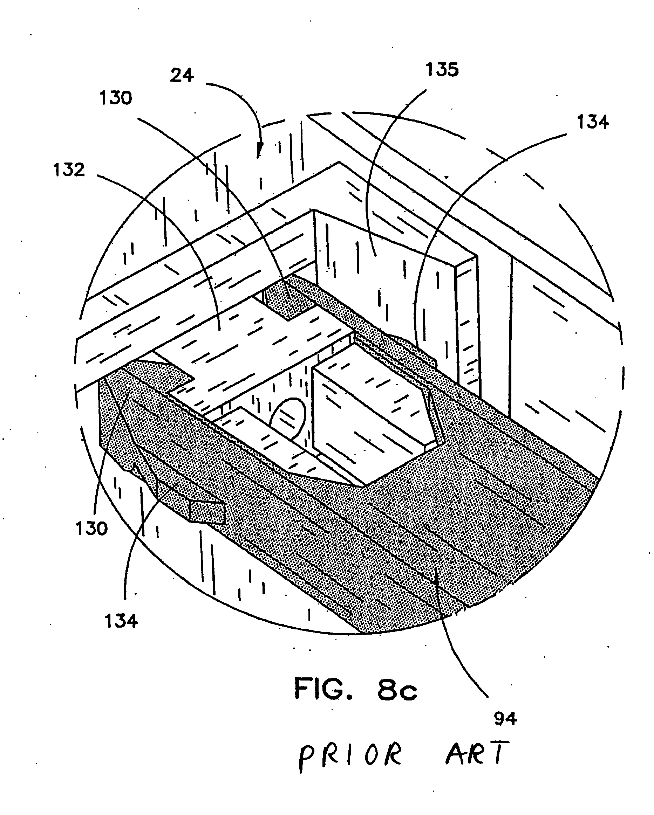

To prevent spring 62 from biasing trigger 24 and housing 22 beyond the home position and to prevent removal of trigger 24 by a user, trigger 24 includes a flange 58 that mates with an upstanding ridge 60. The inventive housing also includes a locking mechanism or "passive lock" that provides sufficient force to prevent the user from accidentally dispensing an article but not too much force to prevent intended dispensing. That is, the passive lock requires initially overcoming a greater force than that provided by spring 62. With reference to Figs. 8a and 8b, trigger 24 defines a home cavity or recess 72 that receives an ear or protrusion 74 formed on back plate 76 (Fig. 4) when the dispenser is in the home position. When the user squeezes the dispenser, sufficient force must be applied to unseat the protrusion 74 from the recess 72. It has been found that a depth of about 0.5 mm for cavity 72 provides a passive lock that provides sufficient force to prevent accidental dispensing. As shown in the detail of Fig. 8b, an inclined recess 80 is provided to guide the protrusion 74 past wall 78 and into the recess 72 during initial assembly.

With reference to Figs. 3, 4 and 6, a cassette 84 that carries stack 34 of test strips 30 is disposed within housing 22. The cassette is preferably made from a three-phase polymer that has moisture-absorbing properties, such as Active-Pak®, available from Capital Specialty Plastics, Auburn, AL, 2AP®, available from Südchemie Performance Packaging Europe, Choisy le Roi, France, and Flotech® "S", available from Grace

Davison, Worms, Germany. As noted above, the reagent materials contained in many test strips degrade when exposed to moisture, and housing them in a desiccant material such as cassette 84 helps address this issue.

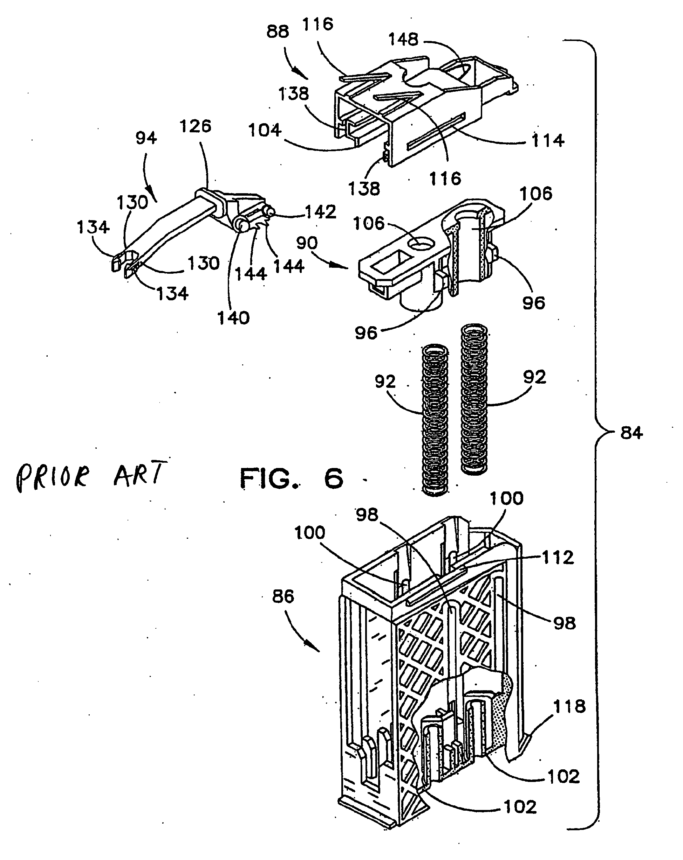

The major components of cassette 84 include back plate 76, which is illustrated in Fig. 4, and hollow body 86, top cap 88, pressure pad 90, pressure pad springs 92 and flexible arm member 94, which are illustrated in Fig. 6. Pressure pad 90 carries the stack of test strips 34 and is movable vertically with respect to hollow body 86. Specifically, pressure pad 90 includes protrusions 96 that are slidingly received in guide slots 98. Two additional protrusions (not shown) extend from the back of pressure pad 90 and are slidingly received into guide slots 100 shown in Fig. 6. The protrusions and guide slots maintain the pressure pad aligned as it advances upward as test strips are dispensed. Once the protrusions reach the top end of the slots, further upward movement of pressure pad 90 is prevented, which would occur when the dispenser is empty. When test strips 30 are present in dispenser 20, upward movement of pressure pad 90 is limited by the uppermost strip abutting against shelves 104 formed in top cap 88. Similarly, the lowermost vertical position of pressure pad 90 occurs when the protrusions reach the bottom of the slots, which occurs when the cassette is filled to capacity with test strips. Cassette 84 preferably holds a range from five to one-hundred fifty of the test strips 34. In the illustrated embodiment, cassette 84 holds fifty test strips.

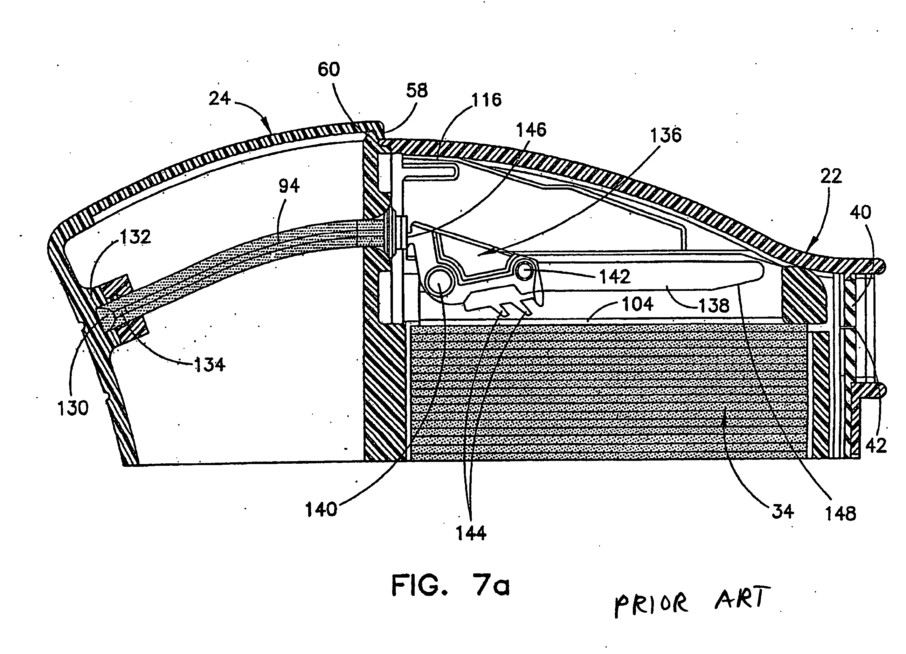

With further reference to Fig. 6, body 86 includes cylindrical spring retention posts 102 on which are received springs 92. Springs 92 extend into cylinders 106 formed in pressure pad 90, and their spring force biases the pressure pad upward as strips are dispensed. Top cap 88 is attached to hollow body 86 by means of anchor stanchions 112 that fit into slots 114. An opening 89 (Fig. 4) is formed between top cap 88 and hollow body 86 to allow a strip to exit the cassette. In the illustrated example, the top cap is formed from polypropylene, although many other materials would be suitable. Spring fingers 116 provide pressure to seat the cassette subassembly in housing 22 as can be seen with reference to Fig. 7a. A toe clip 118 extending from the bottom of body 86 guides the cassette in place during assembly and fits under front rail 120 (Fig. 3) to hold the front of the cassette body 86 in the housing 22. Retention boss 122 retains the lower back half of the cassette subassembly in the housing 22.

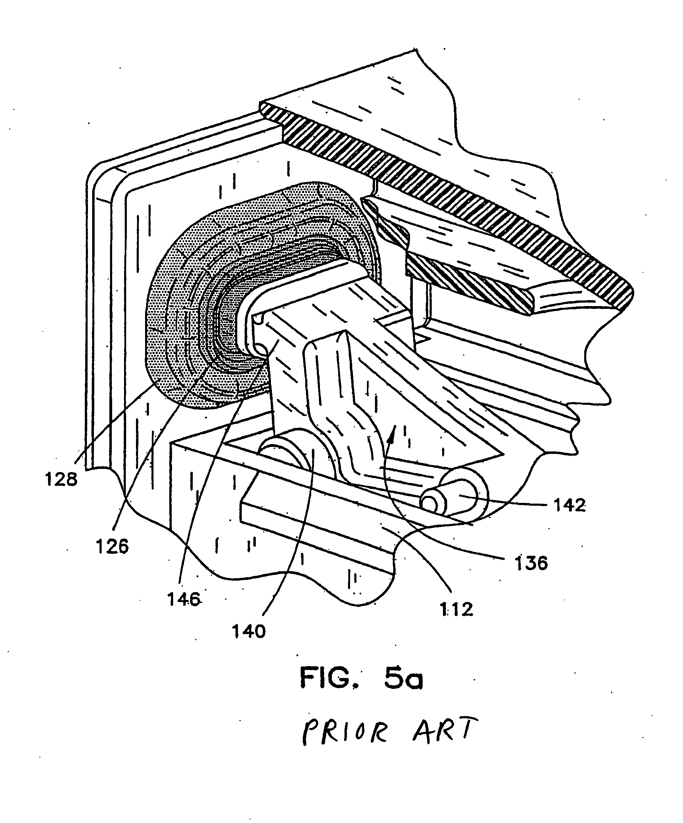

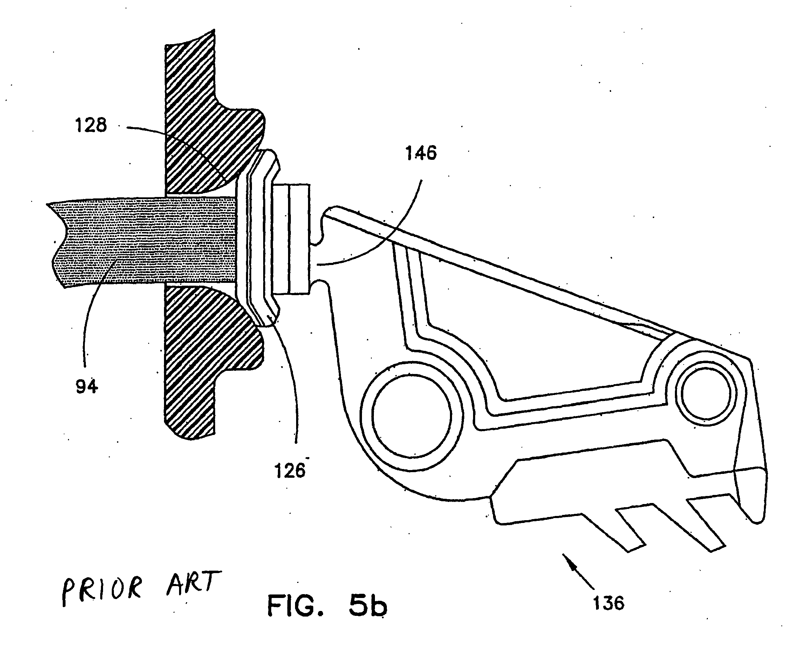

With further reference to Fig. 4, during assembly of dispenser 20, ribs 82 temporarily hold cassette 84 in place within housing 22. Back plate 76 is then welded to the main housing 22 and holds the cassette body 86 within housing 22, which means main housing 22 is essentially sealed from the ambient. The seal is only broken when aperture 124 in back plate 76 is unsealed while a test strip is being dispensed through lip seal 42. However, as shown in Figs. 5a and 5b, flexible arm member 94 includes a sealing member 126 that engages a sealing surface 128 when dispenser 20 is in the home position. Sealing member 126 has an "umbrella" type geometry that circumscribes the flexible arm member and that flexes to conform to the conical recess shape of sealing member 128, thereby forming a tight seal. Sealing member 126 is preferably formed from Santoprene®. When the trigger 24 is squeezed, arm member 94 is actuated and the seal is temporarily broken until dispenser 20 returns to the home position.

In an alternate example (not shown), sealing member 126 is attached to sealing surface 128, such that it stays in place when dispenser 20 is in the home and the dispensed positions. In this embodiment, the sealing member 126 is configured such that flexible arm member 94 passes through the sealing member 126 when the trigger 24 is squeezed. Sealing member 126 circumscribes the flexible arm member 94 but allows arm member 94 to slide through sealing member 126.

As shown in Figs. 6-8, flexible arm member 94 is connected on one end to trigger housing 24 by means of clip fingers 130 that wrap around retention member 132. Ears 134 press against angled sides 135 of trigger housing 24 and prevent clips 130 from disengaging during operation of the dispenser. On its other end, the flexible arm member terminates in a pusher head 136 that is positioned above the test strips and is slidably disposed in groove 138. More particularly, pusher head 136 includes cams 140 and posts 142 extending into grooves 138. The cams, posts and grooves comprise part of a guiding mechanism that moves the pusher head up and down as it reciprocates, as described in more detail below. The pusher head also includes resilient fingers or engagement members 144 that frictionally engage the top surface of the top test strip and slide it out of the dispenser, as discussed in more detail below.

Pusher head 136 is preferably integrally formed with the remainder of flexible arm member 94 and is hingedly connected thereto by a "working hinge" 146. The working hinge provides stronger resistance than a normal "living hinge" but will still flex, allowing the pusher head to pivot or rotate down onto and up and away from the test strips. In the illustrated embodiment, the flexible arm member 94 is formed from polypropylene, but one of ordinary skill in the art could substitute many other flexible materials.

When not in use, the inventive dispenser is positioned in the "home" position depicted in Figs. 1a and 7a. As the user begins to squeeze trigger 24 and housing 22 together, he or she must first overcome the "passive lock" described above. Thereafter, trigger 24 and housing 22 can be squeezed together against the force of spring 62, thereby defining a dispense stroke. Since flexible arm member 94 is coupled to trigger 24, the pivoting movement of the dispenser parts actuates the arm member and causes it to slide through aperture 124. The seal made by umbrella seal 126 is thus broken. Advantageously, however, arm member 94 has a profile that substantially matches that of aperture 124 such that arm member 94 engages the periphery of aperture 124 as it passes through it. A quasi-seal between arm member 94 and aperture 124 thus remains as arm member 94 slides through aperture 124. In other words, even though seal 126 moves away from aperture 124 when the dispenser is activated, because there is a close fit between arm member 94 and aperture 124, the seal there between is not significantly compromised.

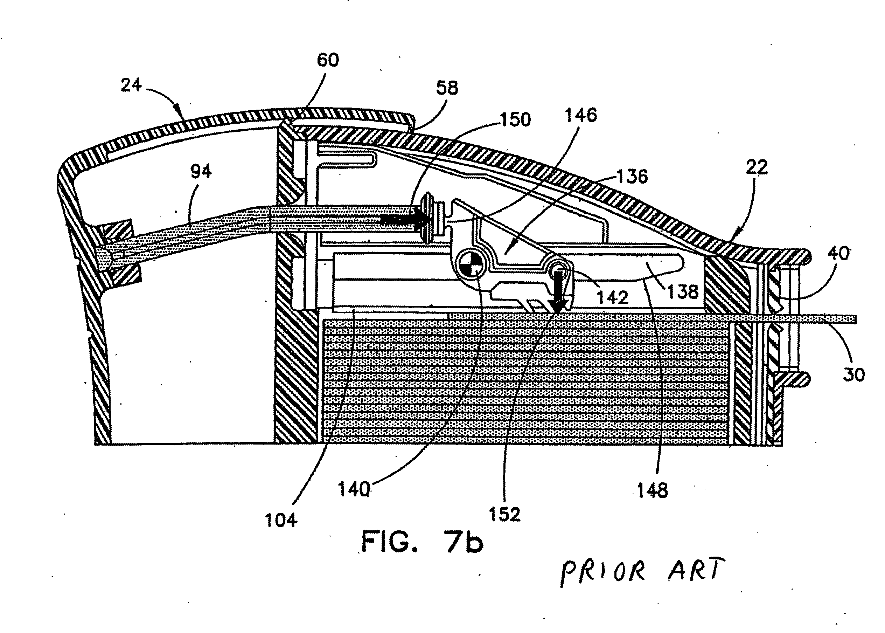

Turning now to Figs. 7a and 7b, this initial movement of the arm member causes pusher head 136 to pivot about cams 140 from a disengaged station spaced away from the stack of strips to an engaged station in which fingers 144 frictionally engage the top test strip. In other words, cams 140 define a pivot axis and the sliding movement in the direction of arrow 150 of arm member 94 as shown in Fig. 7b is translated through working hinge 146 and produces a moment about cams 140. This in turn causes pusher head 136 to pivot down as shown by arrow 152. At the same time, the pusher head slides to the right as shown and the top strip displaces flaps 40 of lip seal 42 as it exits the dispenser. Lip seal 42 maintains a quasi-seal even as strip 30 passes through it. Test strips 30 can be arranged in cassette 84 such that a dosing end or a meter insertion end of the test strip 30 exits first as the pusher head slides to the right.

As shown in Fig. 7b, groove 138 defines an inclined portion 148 at an end thereof. At the end of the dispense stroke, posts 142 are engaged by inclined portion 148, such that pusher head 136 pivots upward and away from the top test strip of the stack, as shown in Fig. 7c. This upward pivoting occurs despite there being a moment about cams 140 through the end of the dispense stroke. Advantageously, since pusher head 136 is pivoted away from the top test strip, the top strip can be easily pulled from dispenser 20 against only a slight frictional force produced by lip seal 42. The flexible arm member flexes into a substantially straight configuration at the end of the dispense stroke as shown in Fig. 7c. The bending or straightening of arm member 94 is due to the fact that the end of arm member 94 that is coupled to trigger 24 moves upward relative to housing 22 as the housing and trigger are squeezed together.

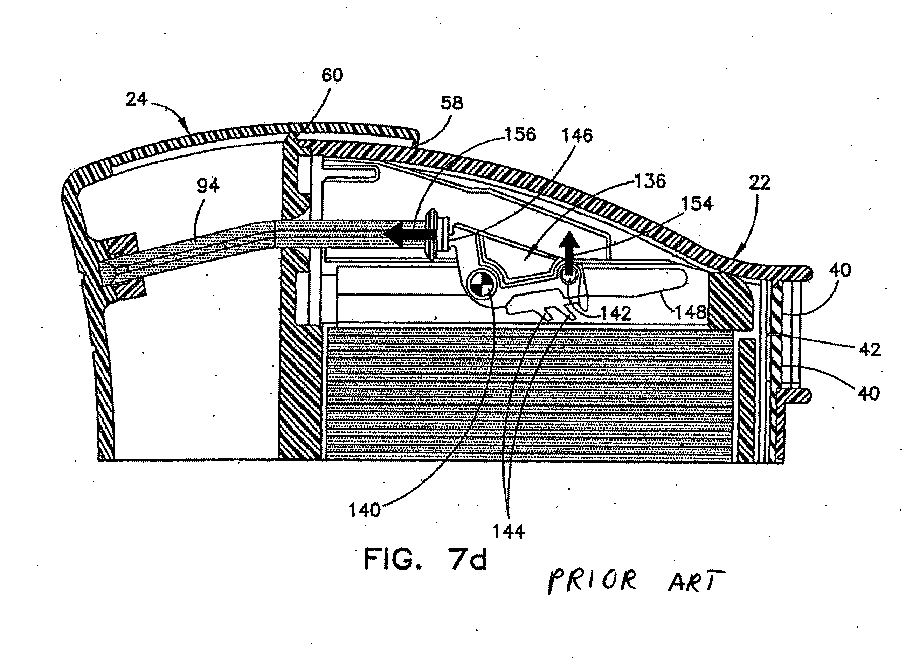

As the user loosens his or her grip and allows spring 62 to return trigger 24 and housing 22 to the home position, an opposite moment is created about cams 140 as shown in Fig. 7d. On the return stroke, the sliding movement in the direction of arrow 156 of arm member 94 as shown in Fig. 7d is translated through working hinge 146 and produces a moment about cams 140 which maintains the pusher head 136 in the disengaged position. Advantageously, even if the user loosens his or her grip before pulling the top strip completely from the dispenser, the pusher head will not engage this top strip on the return stroke. That is, the top strip will not retract on the return stroke simply because the user fails to pull it from the dispenser before releasing the trigger and housing.

It should be appreciated that after the strip is dispensed and the dispenser has returned to the home position, lip seal 42 has automatically returned to its sealed or closed position on its own volition, thereby making it unnecessary for the user to remember to close the dispenser.

Optionally, the dispenser 20 can be integrated with a test meter that reads the test strips. The meter can also receive data from the test strip 30 and/or dispenser 20 and interpret the data. Dispenser 20 can be configured with a radio frequency identification (RFID) tag that stores information about the strips such as lot number, expiration date, type of test strip, among other information. The meter can be configured with an RFID reader which sends a signal to the RFID tag when the dispenser is brought within close proximity of the meter. The meter can thus receive the data that is stored on the RFID tag. The RFID reader and the RFID tag are referred to generally herein as an RFID system.

In one example of an RFID system suitable for use in embodiments of the present invention, the RFID reader includes a transceiver and an antenna that emits electromagnetic radio signals to activate the RFID tag, which includes its own transceiver and antenna. Interrogation signals from the reader activate the tag, causing the tag to send a return signal including the information encoded on the tag.

The RFID system can operate via a technique known as continuous wave backscatter. In this technique, the reader transmits a continuous-wave interrogation signal to the tag, and the tag modulates the continuous wave interrogation signal to produce a backscatter response signal that is transmitted back to the reader. This backscatter response signal includes the information encoded on the tag, such as the lot number, expiration date, calibration data or other information concerning the test strips.

A variety of suitable RFID tag systems are commercially available. Presently, there are three main categories of commercially available RFID systems. There are systems that employ beam-powered passive tags, battery-powered semi-passive tags, and active tags. A beam-powered RFID tag is often referred to as a passive device, in that it derives the energy needed for its operation from the radio frequency energy beamed at it (from the reader). Such a passive tag rectifies the field and changes the reflective characteristics of the tag itself, creating a change in reflectivity (RF crosssection) that is then seen at the reader. A battery-powered semi-passive RFID tag operates in a similar fashion, modulating its RF cross-section in order to change its reflectivity that is seen at the interrogator to develop a communication link. However, the semi-passive RFID tag has a battery to provide the tag's operational power. Finally, in the active RFID tag, both the tag and reader have transceivers to communicate and are each powered by their own batteries.

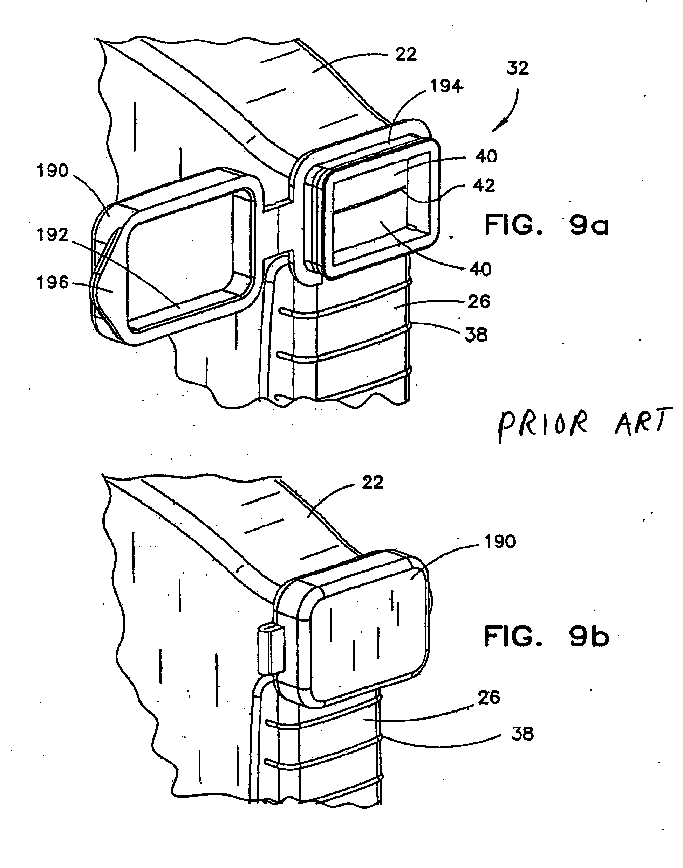

In the illustrated example in Figs. 9a-9b, housing 22 includes a lip seal cover 190 for covering the exit 32. Lip seal cover 190 has an inner surface 192 that defines a receptacle that receives protruding lip 194 of exit 32 by a friction fit engagement. As shown, the lip seal cover 190 is hingedly connected to housing 22. However, other means for making the connection between lip seal cover 190 and exit 32, e.g., a snap-on connection, could be substituted for the hinge connection. When the dispenser is in use, the lip seal cover 190 is pivoted away from the exit 32 by the user applying a finger or thumb to tab 196, which exposes the flaps 40 of lip seal 42, as shown in Fig. 9a. When the dispenser is not in use, the lip seal cover 190 is positioned over the exit 32 to cover the flaps 40 of lip seal 42 as shown in Fig. 9b. Lip seal cover 190 may be formed of any number of materials such as plastics, composites, metals and the like. Advantageously, lip seal cover 190 acts as a dust cover for exit 32 and protects exit 32 from contact damage by items such as keys, coins, cosmetic containers, and the like when the dispenser is carried in a pocket or container having such items.

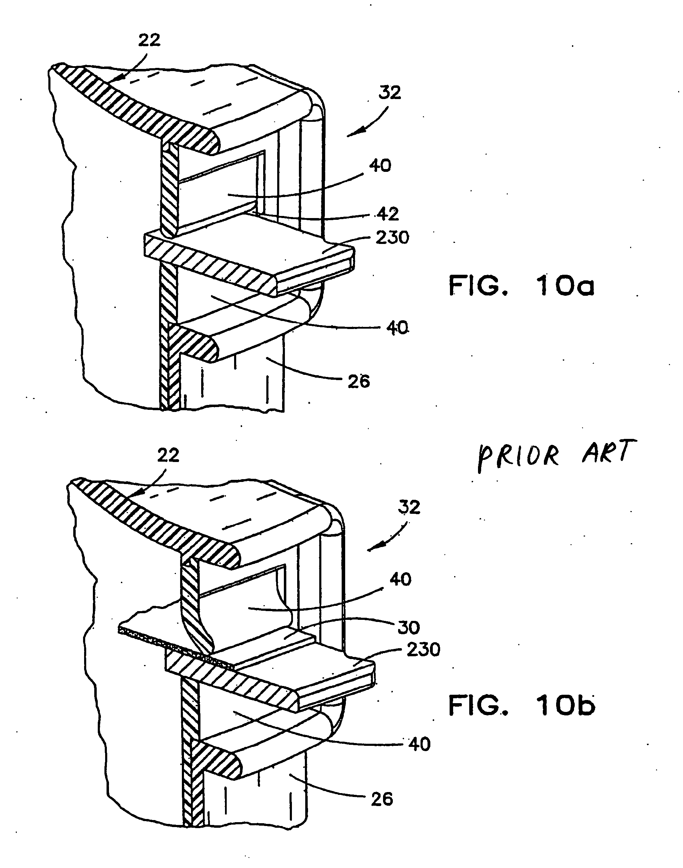

In the example illustrated in Figs. 10a-10b, housing 22 includes a platform 230 inserted between flaps 40 of lip seal 42 and integrally attached to the housing 22. The platform 230 is sized to fit within the lip seal 42 as shown in Fig. 10a. When the dispenser is not in use, the platform 230 inserted between the flaps 40 acts as a plug and forms an airtight seal with the flaps 40. As shown in Fig. 10b, when dispensing the first test strip 30 from the dispenser, the test strip 30 displaces or biases the top flap outwardly as it exits the dispenser. Thereafter, top flap 40 will remain in an outward position, but remains nonetheless biased against platform 230. The platform 230 provides a surface that the test strip 30 can ride on or slide over as the test strip 30 passes through the lip seal 42. It should be appreciated that the dispenser could be configured to dispense test strips between the bottom flap and the platform or the top flap and the platform. Platform 230 may be formed of any number of materials such as plastics, composites, metals or other materials.

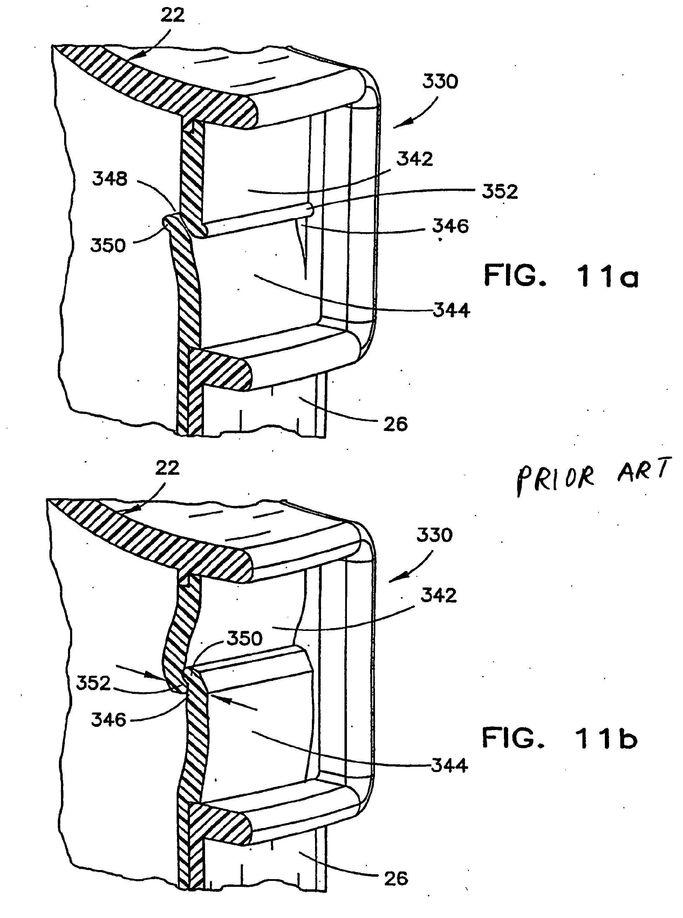

As shown in Figs. 11a-11b, housing 22 includes an exit 330 that can be reconfigured to form an airtight seal. To achieve manufacturing efficiency, it is desirable to form seal 346 in a single step, e.g., by injection molding. It is further desirable to form ribs 38 (Fig. 2) integrally with seal 346. One of the drawbacks of injection molding is that discontinuities such as lip seal 42 require a part in the mold that separates flaps 342 and 344 that are to be formed with the mold. If the seal 346 is to be integrally formed, it will thus include a small gap such as gap 348 illustrated in Fig. 11a between the two flaps.

As formed, seal 346 depicted in Fig. 11a is unsuitable because it is not air-tight. Recognizing that a gap will be present in the originally molded part, the seal 346 shown in Fig. 11a is designed so that it can be "inverted" or "reconfigured" after initially being molded to form an airtight seal in which the two flaps not only contact one another, but are biased together. Specifically, bottom flap 344 is angled to the left or inwardly of the dispenser and includes a nub 350 that points to the left or inside of the dispenser. Flap 342 is preferably angled opposite to flap 344 and also includes a nub 352 that points outwardly or to the right as shown in Fig. 11a. However, after molding, the flexible seal 346 can be reconfigured by inverting the position of the flaps such that flap 342 and flap 344 are biased against each other to form an airtight seal as shown in Fig. 11b. For example, in Fig. 11a, a force can be applied to the top flap 342 to push the top flap 342 to the left and over the bottom flap 344 and/or a force can be applied to the bottom flap 344 to push the bottom flap 344 to the right and under the top flap 342. Alternatively, the airtight seal shown in Fig. 11b can be formed by ejecting the first test strip from dispenser 20, during which the test strip will push flap 344 to the right, past flap 342. When the test strip is fully removed from the dispenser, the configuration shown in Fig. 11b will be achieved.

Since flap 342 as molded tends to point to the right and flap 344 as molded tends to point to the left, inverting the seal to the configuration shown in Fig. 11b creates an advantageously airtight seal in which the flaps are pressed against one another. As shown reconfigured in Fig. 11b, the top flap 342 is displaced to the left of bottom flap 344. Further, the top flap 342 and the bottom flap 344 are biased together as shown by the arrows in Fig. 11b. When dispensing a test strip, the test strip displaces or biases apart the bottom flap 344 from the top flap 342 as it passes between the flaps and exits the dispenser. As described with reference to other embodiments, seal 346 can be formed from Santoprene® or another elastic material that allows top flap 342 and bottom flap 344 to flex or bend. Additionally, the flexible seal 346 can be covered with a cap (see Fig. 9a).

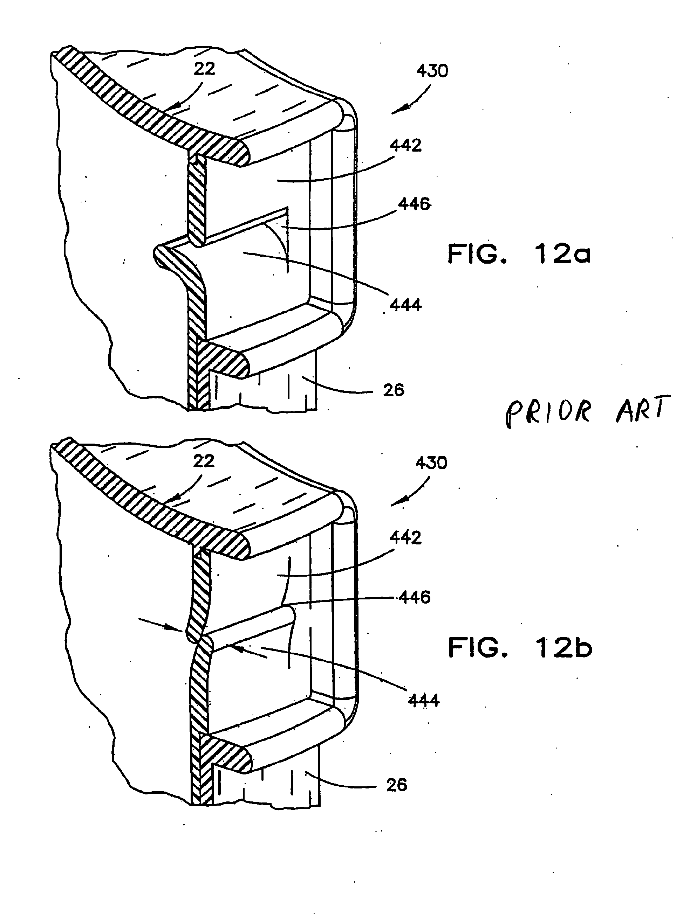

In the example illustrated in Figs. 12a-12b, housing 22 includes an exit 430 that can be reconfigured to form an airtight seal. As shown in Fig. 12a, exit 430 as molded includes top flap 442 and bottom flap 444 that form a flexible seal 446. The bottom flap 444 is positioned initially to the left of top flap 442. In this configuration, top flap 442 and bottom flap 444 are substantially in the same position as when the flexible seal 446 was formed or molded. The flexible seal 446 can be reconfigured by inverting the positions of the flaps such that flap 442 and flap 444 are biased against each other. For example, a force can be applied to the bottom flap 444 shown in Fig. 12a to push or pull it to the right and under the top flap 442.

As shown reconfigured in Fig. 12b, the top flap 442 is displaced to the left of bottom flap 444. Further, the top flap 442 and the bottom flap 444 are biased together as shown by the arrows in Fig. 12b. When dispensing a test strip, the test strip displaces or biases apart the bottom flap 444 from the top flap 442 as it passes between the flaps to exit the dispenser. Top flap 442 and bottom flap 444 can be formed from Santoprene® or another elastic material that allows top flap 442 and bottom flap 444 to flex or bend.

Further, top flap 442 and/or bottom flap 444 can be integrally formed with ribs 38 (see Figs. 1 and 2). Additionally, the flexible seal 446 can be covered with a cap (see Fig. 9a).

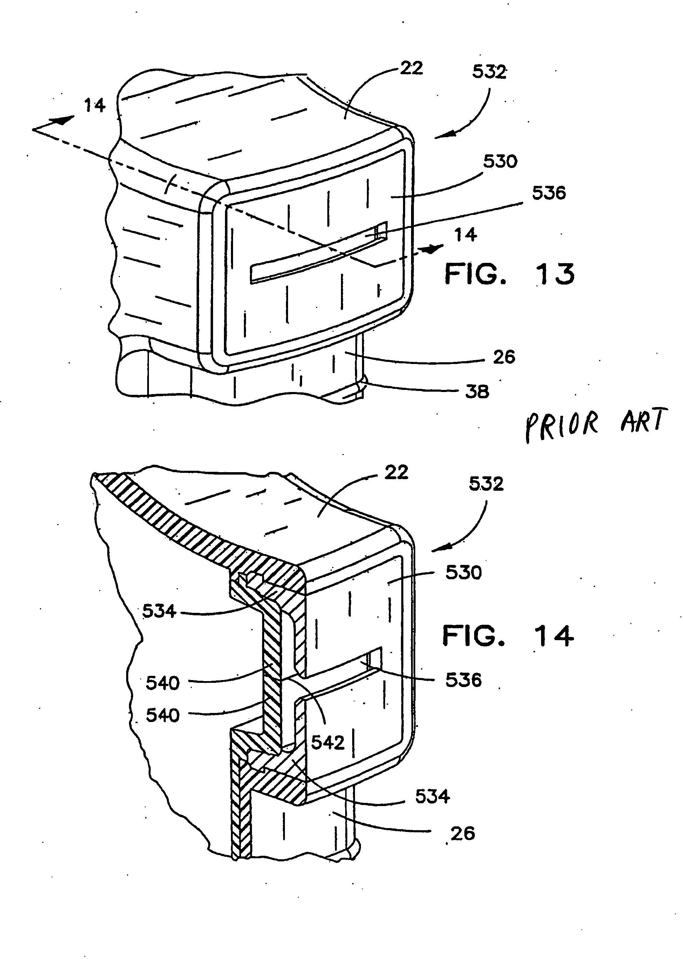

In the example illustrated in Figs. 13-14, housing 22 includes a cap 530 for covering exit 532. Cap 530 has an outer periphery that substantially matches that of exit 532 but is slightly smaller, such that cap 530 fits snugly within the periphery defined by exit 532. Cap 530 includes an opening 536 from which test strips 30 are dispensed. Cap 530 may be formed of any number of materials such as plastics, composites, metals or other materials. Cap 530, like lip seal cover 190, also acts as a dust cover for exit 532 and protects exit 532 from contact damage by foreign objects.

Exit 532 includes flaps 540 that form a flexible seal 542 as shown in Fig. 14. Further, biasing members 534 are wedged between the housing 22 and the flaps 540 to reconfigure the flexible seal 542 as shown in Fig. 14 such that the biasing members 534 bias the flaps 540 together to form an airtight seal. That is, as cap 530 is installed into dispenser 20, wedge-shaped biasing members 534 are inserted between the housing 22 and seal 542 such that biasing members 534 squeeze the flaps 540 together. As shown, biasing members 534 are integrally formed with cap 530. When dispensing a test strip, the test strip displaces or biases apart the flaps 540 as it passes between the flaps 540 to exit the dispenser through opening 536. Flaps 540 can be formed of Santoprene® or another elastic material that allows the flaps 540 to flex or bend. Also, it should be appreciated that the embodiment shown in Figs. 9a-9b can also be configured to include biasing members that squeeze the flaps together.

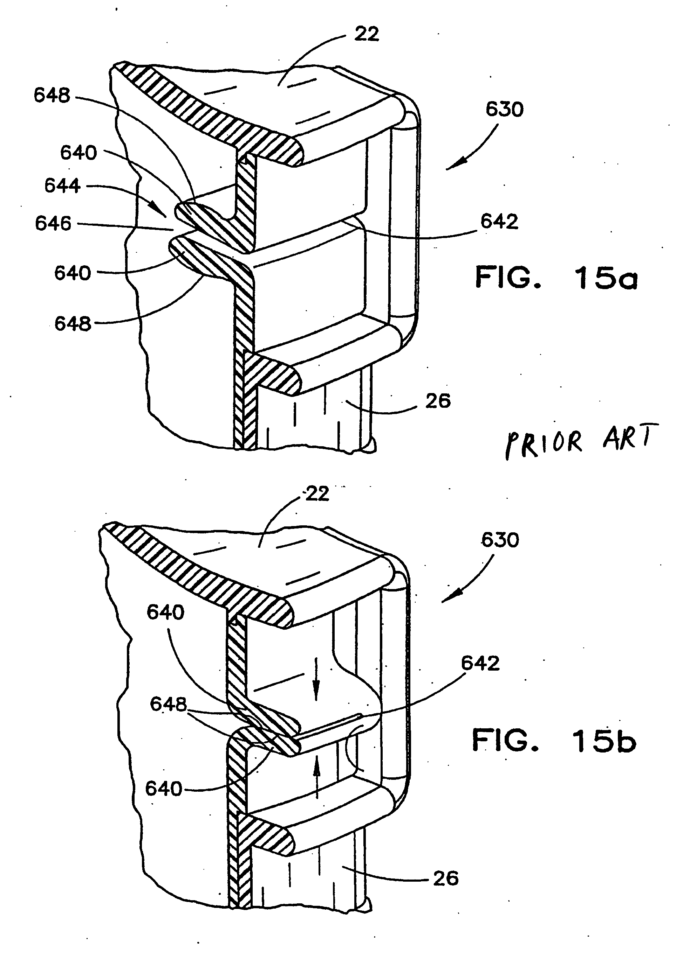

As shown in Figs. 15a-15c, housing 22 includes an exit 630 that can be reconfigured to form a substantially or completely airtight "duckbill" seal. Exit 630 is shaped as a duckbill with walls 640 that form an elongated channel 644, as illustrated in Fig. 15a. The walls 640 point to the left in Fig. 15a. In this configuration, walls 640 are substantially in the same position as when the flexible seal 642 was formed or molded. As described elsewhere, since the seal 642 is preferably integrally formed by injection molding, the walls 640 define a gap 646 therebetween when the part leaves the mold from which it is made. However, the gap 646 can be eliminated by inverting or turning the duckbill seal 642 inside out, as can be appreciated by comparing Figs. 15a and 15b.

The curved or profiled outer sides 648 of walls 640 shown in Fig. 15a become the inner walls of the seal shown in Fig. 15b, in which the walls 640 are biased together and form an air-tight seal, as indicated by the arrows in Fig. 15b. The profiled shape of walls 640 shown in Fig. 15a enhances the bias between walls 640 when the seal 642 is turned inside out to form the structure shown in Fig. 15b.

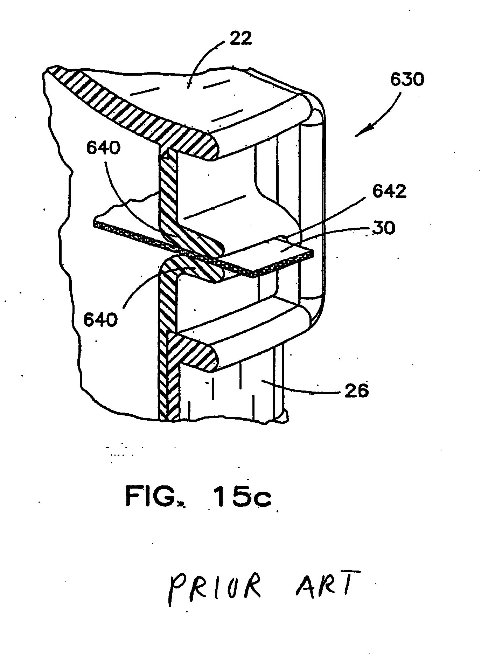

When dispensing test strip 30 as shown in Fig. 15c, the test strip 30 displaces or pushes apart the top wall from the bottom wall as it passes between the walls 640 to exit the dispenser. Walls 640 can be formed from Santoprene® or another elastic material that allows the walls 640 to flex or bend. Further, walls 640 can be integrally formed with ribs 38 (see Figs. 1 and 2). In addition, the flexible seal 642 can be covered with a cap (see Fig. 9a).

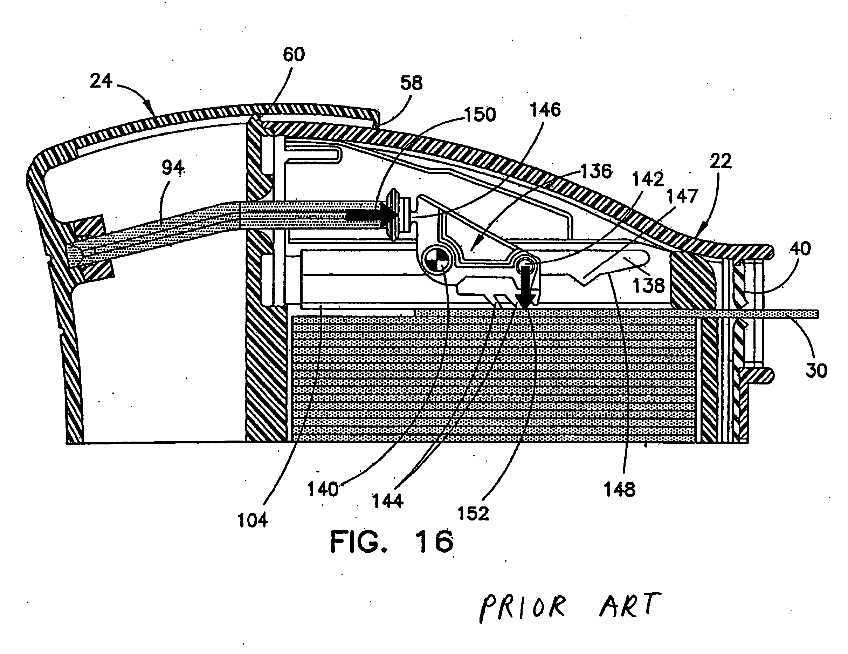

The dispenser can be configured to facilitate inserting a strip into a meter without the need for the user to have to touch a strip. For example, in Fig. 16, the stack of test strips is loaded into the dispenser such that the "meter insertion end" of the test strips exits the dispenser first upon dispensing. (This is opposite to that shown in Fig. 2.) Further, the dispenser is configured with an additional "detent" position between the home and dispense positions described above, which is used to hold the test strip in place after the end of it has extended from the dispenser.

With further reference to Fig. 16, groove 138 defines a notch or detent 147 such that posts 142 engage the detent 147 as the pusher head 136 slides to the right as the user squeezes the trigger and housing together. The detent 147 is positioned such that the posts 142 are guided into it as the dispenser is actuated. This happens after test strip 30 has passed partially through lip seal 42 and has a portion thereof extending from the dispenser as shown. The user experiences a tactile sensation when the forward movement of pusher head 136 stops as the posts 142 engage the detent 147. In this detent position, the fingers 144 remain frictionally engaged with the top test strip. The fingers 144 captively hold the test strip 30 such that the meter insertion end of the test strip 30 extends from the exit 32 of the dispenser.

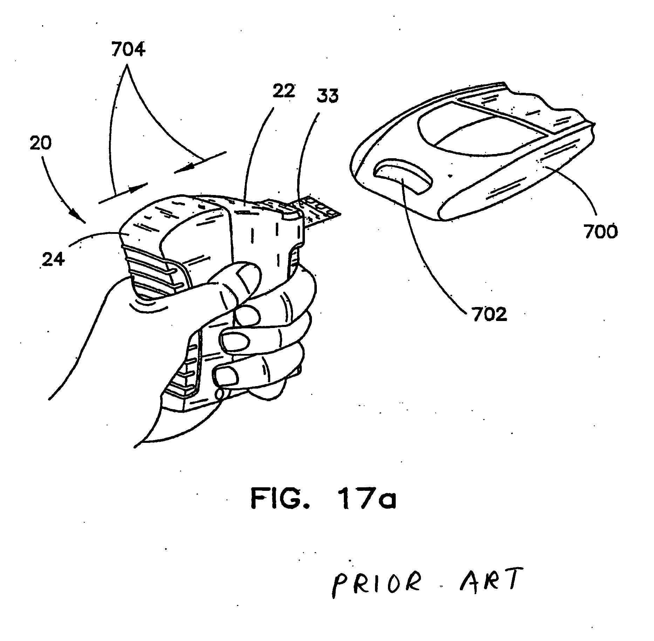

In this intermediate or detent position, the user can "dock" dispenser 20 to a test meter to receive the test strip 30. As shown in Figs. 17a-17c, test meter 700 has an opening 702 to receive the meter insertion end 33 of test strip 30. In Fig. 17a, the user has squeezed trigger 24 and housing 22 together in the direction of arrows 704 to overcome the "passive lock" described above and has continued to squeeze trigger 24 and housing 22 together to the detent position. The detent position is signaled to the user through a tactile sensation produced by posts 142 engaging detent 147, at which point the forward movement of pusher head 136 stops. In this embodiment, the meter insertion end 33 of the test strip 30 extends from exit 32 of dispenser 20 and fingers 144 remain frictionally engaged with the test strip.

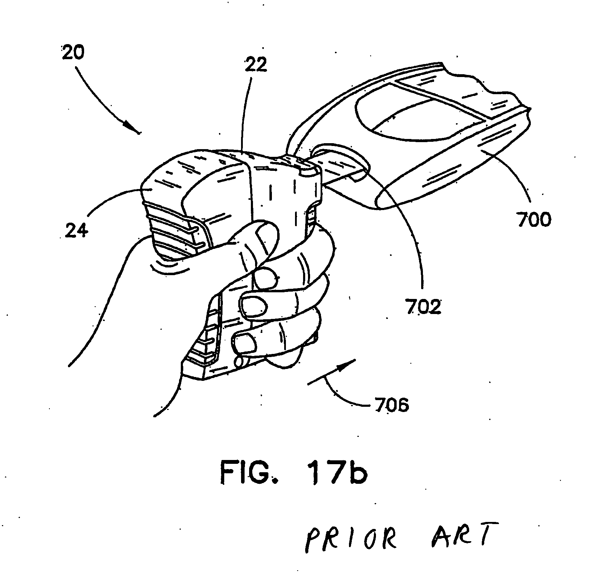

The user then aligns the end 33 of the test strip 30 with the opening 702 and moves the dispenser in the direction of arrow 706 in Fig. 17b so that the meter insertion end of the test strip is inserted into opening 702 of test meter 700. Preferably, the test meter 700 engages and captively holds the end of the test strip after it is inserted to the required depth.

At this point, the meter and dispenser are in close proximity. If the dispenser is configured with the optional RFID tag noted above, and meter 700 includes an RFID reader, the meter will download data from the RFID tag. Such data may include calibration data, expiration date and the like for the strips housed in dispenser 20. In many traditional test strip vials, this information is included in a memory chip that is packaged with the vial. The memory chip must be inserted into the meter by the user before using the strips in a given vial. The RFID tag disclosed herein can avoid the need for these memory chips and the need for the user to have to insert them into the meter or otherwise code the meter for the test strips.

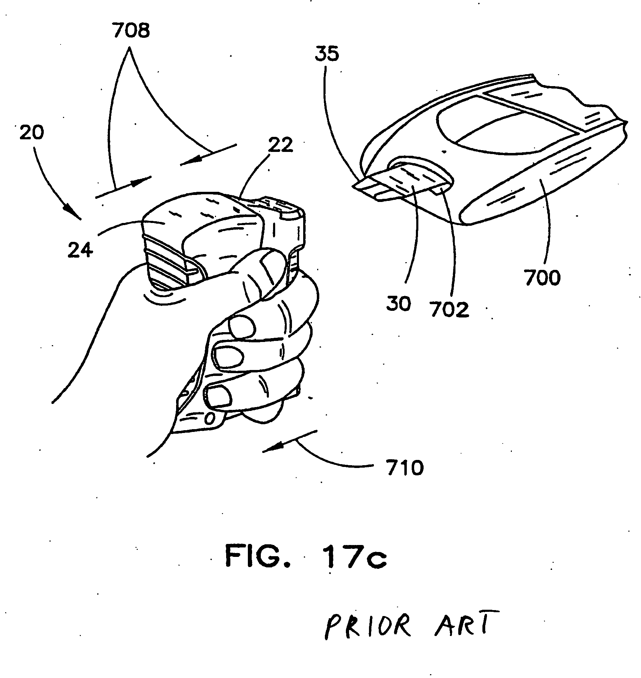

After the dispenser and meter are "docked" as shown in Fig. 17b, the user then "releases" the test strip from the dispenser. With reference to Fig. 17c, this is done by fully squeezing the trigger 24 and the housing 22 together in the direction of arrows 708 to arrive at the "dispensed" position described above with respect to other embodiments. In the dispensed position, the fingers lift from the strip, thus releasing it. The dispenser can then be pulled away from the meter as shown by arrow 710 while leaving test strip 30 inserted in opening 702 of test meter 700, as shown in Fig. 17c. A dosing end 35 is thus protruding from the meter and is ready to receive a fluid sample.

The range of communication for RFID tags in general depends upon the transmission power of the reader and of the tag, with a greater range requiring greater transmission power. RFID reader power for proper operation of passive RFID systems is a function of distance, antenna sizes, frequency, and orientation. Most RFID systems are intended for near-field applications. Distances between readers and tags are normally on the order of millimeters. An example of a simplified equation for inductive based passive devices that help illustrate the impact of distance changes is as follows:

$$H = (I\,N)/(2\,r\,(\,1 + (d^2/r^2))^{1.5}$$

where H = magnetic field intensity, I = current through reader antenna coil, N=number of turns on the reader antenna coil, r=radius of the reader antenna coil, d=distance between the center of the reader antenna coil & the center of the tag antenna coil.

However, these equations are typically useful only when the d is of the same order of magnitude as r (i.e., near-field).

Because a passive tag derives its power from the interrogation signal of the reader, the transmission power is dependent on the transmission power of the reader. To reduce the power demands on the reader and prolong its battery life (and thereby the battery life of the reader), RFID systems used in the present invention can be configured to operate in a relatively short transmission range (on the order of inches), thus reducing transmission power requirements. For example, in one embodiment the power of the interrogation signal is less than about 1 watt and in other embodiments is less than about 50 milliwatts. In these or in other forms, the overall power consumed by the reader to interrogate and read the RFID tag in one embodiment is less than about 325 milliwatts, and in other embodiments is less than about 30 milliwatts.

Limiting the RFID communications sessions to situations when the tag and reader are in close proximity further conserves battery life. For example, an RFID communication protocol may be employed that limits communications attempts from the reader (i.e. the sending of interrogations signals) to situations where a tag is present to be read. One such protocol places the reader in sleep mode until a test strip is inserted into the meter. Insertion of the test strip activates the reader to send out its interrogation signal and to look for the response from the RFID tag. The response comes in a matter of milliseconds, and once the information from the RFID tag has been received at the meter, the reader goes back into battery conservation/sleep mode. Triggering the RFID tag interrogation signal based on the docking of a test strip into a meter serves to assure that communication is only attempted when the meter and the dispenser are in sufficiently close proximity.

FIG. 18 illustrates a dispenser 900 according to an embodiment of the present invention. Like dispenser 20 described above, dispenser 900 is constructed to sequentially dispense test strips 30 out of an opening 932, and as illustrated has strip 33 extending from opening 932 so as to be ready to dock with meter 700. Dispenser 900 includes an RFID tag 999 snap fit into a receiving pocket 998 near opening 932 such that, when docked with meter 700 in the manner shown in Fig. 17a, the absolute distance between tag 999 and the reader in meter 700 is minimized. Depending on the precise location of the RFID reader in meter 700, similar proximity can be achieved by locating tag 999 above or to the side of opening 932. In still other variations, and particularly where close proximity is not needed, tag 999 can be located anywhere in dispenser 900.

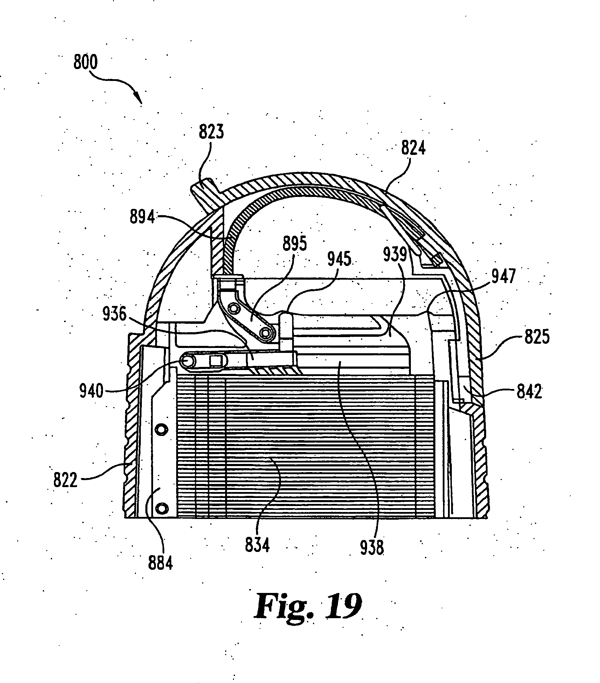

Aside from the inclusion of receiving pocket 998 for tag 999, the primary difference between dispensers 20 and 900 relates to the manner of activation by the user. More specifically, while each are constructed for one handed operation, dispenser 900 employs a trigger 924 rotatable about the housing 922 for activation whereas dispenser 20 utilizes squeeze action. As described more fully below with respect to Figs. 19-23, the trigger 924 can be operated with the user's thumb while holding the dispenser 900. Such a construction provides a compact and user friendly device that, like the dispenser 20, is particularly suitable for operation by patients with limited dexterity in the hands.

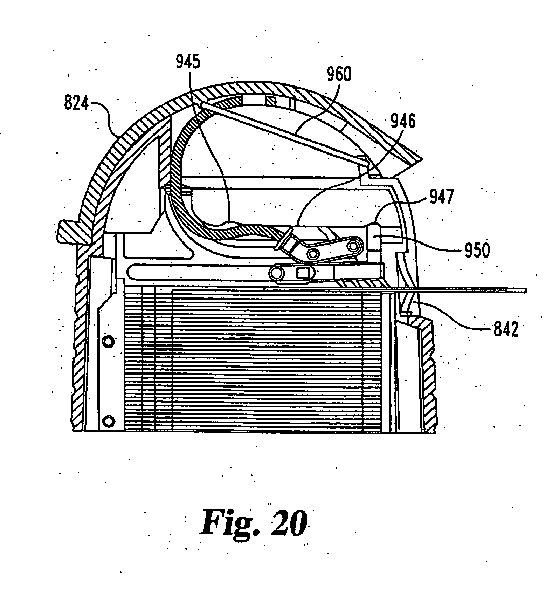

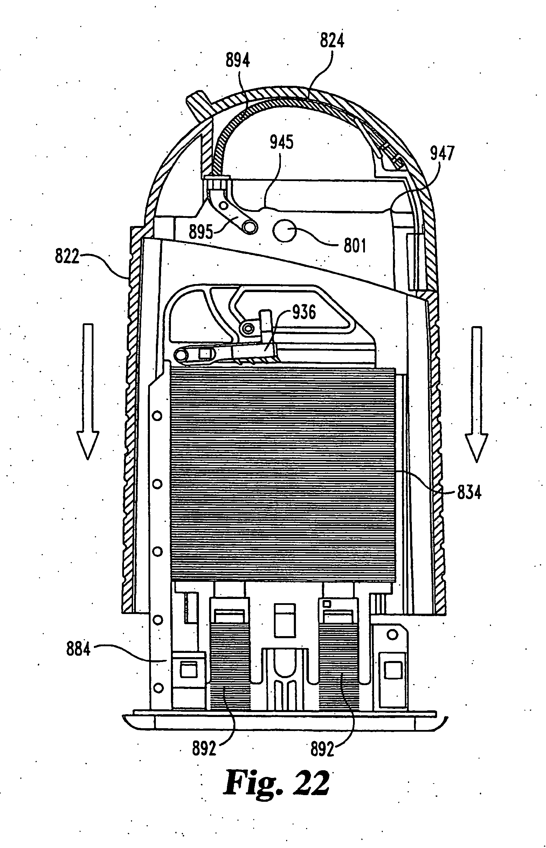

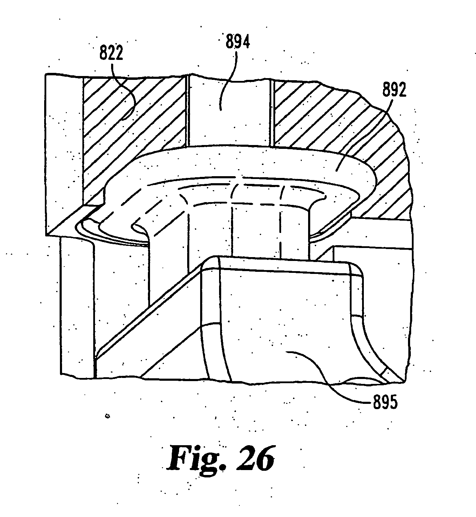

Referring now to Figs. 19-26, the activation of the trigger will be described in connection with dispenser 800, which is a variation of dispenser 900 without the RFID tag 999 or the receiving pocket 998. Dispenser 800 includes two main components: a main outer housing 822 and an inner cassette 884 that holds a stack 834 of test strips. A trigger 824 is pivotally mounted to the housing 822 and is coupled to a flexible arm 894. The housing 822 receives the cassette 884, as indicated by the downward arrows showing assembly direction in FIG. 22, and a rigid portion 895 of flexible arm 894 couples via a yoke type coupling to pusher head 936. Pusher head 936 is slideably contained in cassette 884 above the test strip stack 834, and head 936 includes a friction pad with flexible fingers 936 for frictionally engaging the uppermost test strip in the stack 834. A pair of guide slots 938, 939 in cassette 884 receive corresponding guide followers 940, 891 on pusher head 936 and the rigid portion 895 of flexible arm 894, respectively. In use, a user grips the dispenser and depresses the outer surface of trigger 824, for example at a frictional element such as ridge 823, with his thumb to pivot the trigger 824 about the axis defined by pin 801. This moves trigger 824 from the home/closed position (Fig. 19) to the dispensed/open position (Fig. 20) and causes arm 894 to cause pusher head 936 to slide from left to right in Figs. 19 and 20.

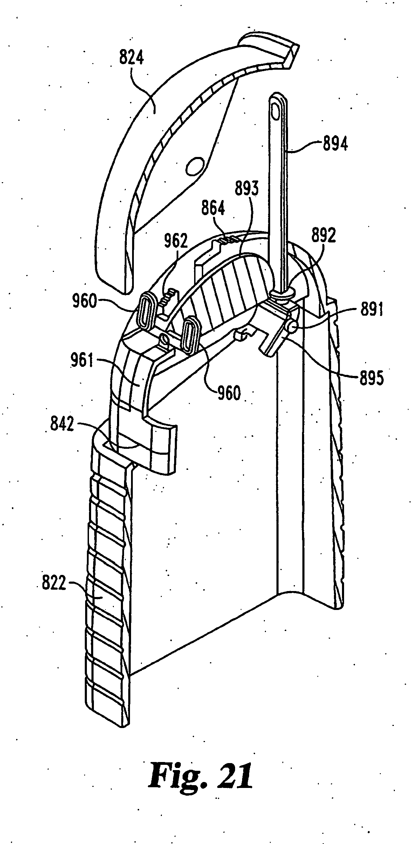

As shown in Figs. 19 and 20, because the pivotal connection is made between a central lower portion of the trigger 824 and a central upper portion of the housing 822, the movement of trigger 824 and housing 822 relative to one another maintains the nesting relationship between them from the home position to the dispense position and back again. Trigger 824 has a profile that substantially matches that of housing 822 but is slightly larger, such that the inner surface of trigger 824 defines a receptacle for housing 822. As shown more clearly in Fig. 21, trigger 824 defines an arcuate inner wall 854 that has an arcuate profile similar to that of outer wall 856 defined by housing 822. Thus, when trigger 824 is rotated from a home position to a dispense position, housing 822 is substantially continually nested within trigger 824 while an article 33 is expelled partially from dispenser 800. Thus, trigger 824 is rotatable about the housing 822.

In the home position of Fig. 19, a pusher pad pressure control follower 950 of pusher head 936 is received in detent 945 of a pressure control cam rail 946 of housing 822. As head 936 slides to the right, cam rail 946 applies downward pressure, pressing the fingers 937 of head 936 against the top strip of stack 834 to achieve a secure frictional coupling. This frictional coupling forces the top strip out lip seal 842 as head 936 completes its operative stroke. When follower 950 of head 936 reaches detent 947 (the dispensed position of Fig. 20), the downward pressure is released, reducing the frictional engagement and allowing easy withdrawal of the strip from the dispenser 800. As head 936 returns to the home position, the next strip in stack 834 is biased upward by cassette springs 892 to be in position for the next dispensing. Accordingly, the cam rail 946 provides a biasing surface that follower 950 slides along to apply downward pressure on the pusher head 936 during its dispense stroke, and recesses 945, 947 in the biasing surface serve to reduce this downward pressure when the pusher head 936 is in its home and dispensed positions.

During operation, and as shown in Figs. 19 and 20, flexible arm 894 follows a C-shaped or arcurate path and is at least partially guided therein by guide rail 893 (see Fig. 21). When sized as a handheld device, this arcuate path of arm 894 with be of relatively small size, for example portions having a radius of curvature less than 3, 2 or 1 inch. Likewise, to achieve a compact device, a substantial portion of the arcuate path, including, therefore, a substantial portion of the arm 864, is maintained above the stack 834 (per the views of Figs. 19 and 20) during the operative stroke.

As shown more particularly in Fig. 21, the trigger 824 is biased to be in the home, or closed position of Fig. 19 by a pair of elastic bands 960. Bands 960 are secured at one end (to housing 822) and have their open ends looped around one or more catches 963 formed on the inside of trigger 824. In one embodiment, bands 960 are formed as a part of elastic member 961 which is a unitary elastomeric piece (e.g. Santoprene ®) that defines the lip seal 842 at one end and the bands 960 at another. In other embodiments, bands and lip seal are separate pieces.

As depicted, there are four fingers 937 on the pusher head 936, each angled in the direction of dispensing. These fingers 937 are formed of a resilient material, such as rubber or polypropylene, and are rigid enough to remain angled in the direction of dispensing during the dispensing stroke, but are flexible enough to "give" during the return stoke. Thus, the bias of the bands 960 is sufficient to overcome the drag force of the fingers 937 against the top strip in the stack and to return the pusher head 936 to the home position (Fig. 19).

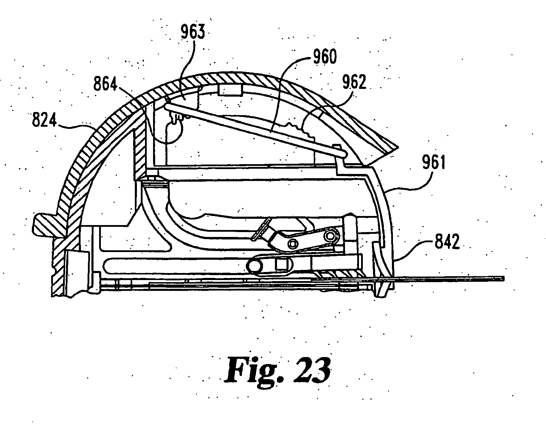

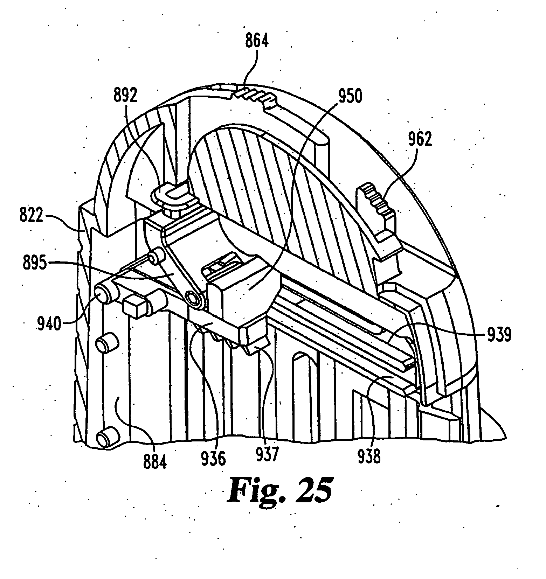

Referring now to Fig. 23, in one embodiment, catches 963 also comprise a series of ratchet members 864 that interact with first and second pawls 962, 864 (also shown in perspective in Figs. 21 and 25) to indicate when the trigger 824 is in the home position or dispense position, respectively. In operation, when trigger 824 is in the home position, the ratchet members 864 of catches 963 engage first pawls 962 to yieldingly retain the trigger 824 in the home position in order to reduce the chances that trigger 824 will be accidentally opened. When trigger 824 is opened, the ratchet members 864 of catches 963 engage second pawls 864 to yieldingly retain the trigger 824 in the dispense position against the restoring force of bands 960.

In the closed position, portion 825 of trigger 824 is covering and protecting the exit defined by lip seal 842.

Thus, by helping to keep trigger 824 closed, first pawls 962 are helping to protect and maintain the integrity of the seal 842. Optionally, pawls 962 and/or bands 960 bias portion 825 of trigger 824 to contact and/or exert pressure on seal 842 to further help preserve its integrity. In other embodiments, trigger 824 covers but does not directly contact seal 842.

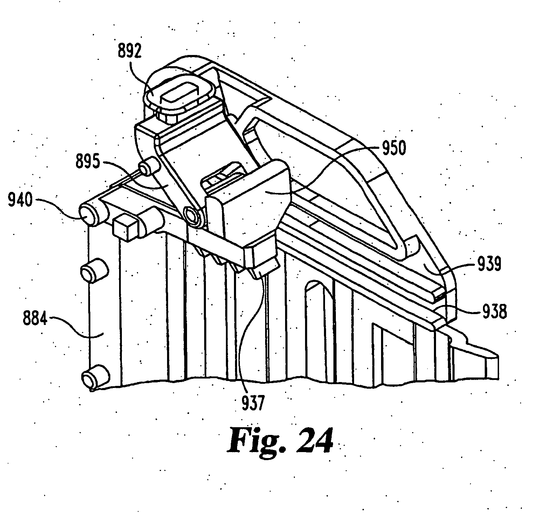

In one mode of construction, at least the upper portion of the inner cassette 884 is constructed in two pieces that are snapped and/or sealed together around the pusher head 936. Figs. 24 shows a perspective view of the pusher head 936 received in one half of the upper part of cassette 884, and Fig. 25 shows the Fig. 24 arrangement with the outer housing 882 attached over the cassette. The flexible arm 894 is not shown in Figs. 24 and 25, but it is to be understood that, as depicted in Figs. 21 and 22, the rigid portion 895 may be attached to the flexible arm 894 before being coupled to the pusher head 936.

The cassette 884 and upper housing 822 cooperate to form a substantially airtight and/or moisture proof enclosure around the test strip stack 834, which serves to increase the shelf life of the test strips. The primary means for moisture in the air to access stack is via the path traveled by the flexible arm 894. As shown in the enlarged view of FIG. 25, an umbrella seal 892 is provided along arm 894 near the rigid portion 895 to form a seal along this path when the pusher head 936 is in its home position (Fig. 19). It is to be understood that umbrella seal 892 mates with a corresponding recess in housing 822 in the same manner that umbrella sealing member 126 engages sealing surface 128 in device 20 above (see Figs. 5a and 5b). Because the devices (20, 800) are in their home positions a majority of the time, this sealing from the external environment can substantially increase the shelf life of the test strips. A dessicant material is optionally provided at the bottom of the stack between cassette springs 892 for similar purposes.

While the operation of the second arrangement of a dispenser has been described with respect to the particular embodiment of the figures, it is to be appreciated that a number of variations could also be employed. For example, other mechanisms for translating the pivoting motion of the trigger to linear motion of a test strip that do not rely on a flexible arm can be employed as would occur to those of skill in the art. For example a slotted cam arrangement could be coupled to the trigger to linearly drive an ejector pad when the top is flipped. In another example, a set of gears could be used to translate the motion of the trigger into a linear driving force to eject a test strip.

While preferred embodiments incorporating the principles of the present invention has been disclosed hereinabove with reference to figures 18-26, the present invention is not limited to the disclosed embodiments. The scope of the invention is defined by the claims.

**Claims**

1. A dispenser for test strips (800), comprising:

   a housing (822) containing a stack of the test strips (834) and defining an exit through which the test strips are dispensed;
   an actuator for moving a first test strip (33) in the stack at least partially through the exit when the actuator is moved from a home position to a dispensed position; and
   a trigger (824) for moving the actuator from the home to the dispensed position;
   wherein the trigger (824) has a first portion adapted to be manually activated by a user and **characterised in that** the trigger (824) has a second portion (825) that covers the exit when the actuator is in the home position.

2. The dispenser of claim 1 wherein the exit (932) comprises a flexible seal (842).

3. The dispenser of claim 2 wherein the trigger (824) is biased to contact and apply pressure to the flexible seal (842) when the actuator is in the home position.

4. The dispenser of claim 1 wherein the actuator includes a pusher head (936) slidably disposed in the housing (822) and a flexible arm (894) coupled to the trigger (824) and the pusher head (936), the pusher head (936) having an engagement member for engaging a first test strip (33) in the stack to move the first test strip at least partially through the exit.

5. The dispenser of claim 1 further comprising a guide assembly (893) that biases the actuator against the top test strip (33) of the stack when the actuator is between its home and dispensed positions.

6. The dispenser of claim 5 wherein the actuator comprises a pusher head (936) having a plurality of fingers (937).

7. The dispenser of any of the preceding claims, wherein the housing (822) receives an inner cassette (884) that holds a stack (834) of test strips.

8. The dispenser of any of claims 4 to 7, wherein the trigger (824) is pivotally mounted to the housing (822) and is coupled to the flexible arm (894).

9. The dispenser of any of claims 4 to 8, wherein the flexible arm (894) comprises a rigid portion (895).

10. The dispenser of any of claims 7 to 9, comprising a pair of guide slots (938, 939) in the cassette (884) that receive corresponding guide followers (940, 891) on pusher head (936) and the rigid portion (895)

11. The dispenser of any of claims 4 to 10, wherein a pusher pad pressure control follower (950) of the pusher head (936) is received in a detent (945) of a pressure control cam rail (946) of the housing (822).

12. The dispenser of claim 11, wherein the cam rail (946) provides a biasing surface that follower (950) slides along to apply downward pressure on the pusher head (936) during its dispense stroke.

13. The dispenser of claim 12, further comprising recesses (945, 947) in the biasing surface that reduce the downward pressure when the pusher head (936) is in its home and dispensed positions.

14. The dispenser of any of the preceding claims, wherein the trigger (824) is biased to be in the home or closed position by a pair of elastic bands (960).

15. The dispenser of any of claims 7-14, wherein the cassette (884) and upper housing (822) cooperate to form a substantially airtight and/or moisture proof enclosure around the test strip stack (834).


**Patentansprüche**

1. Spender (800) für Teststreifen mit einem Gehäuse (822), das einen Stapel (834) der Teststreifen enthält und einen Auslass definiert, durch den die Teststreifen ausgegeben werden, einem Stellglied zum Bewegen eines ersten Teststreifens (33) im Stapel mindestens teilweise durch den Auslass, wenn das Stellglied von einer Ausgangsposition in eine Ausgabeposition bewegt wird, und einem Auslöser (824) zum Bewegen des Stellglieds von der Ausgangs- in die Ausgabeposition, wobei der Auslöser (824) einen ersten Abschnitt hat, der geeignet ist, von einem Benutzer manuell aktiviert zu werden, **dadurch gekennzeichnet, dass** der Auslöser (824) einen zweiten Abschnitt (825) hat, der den Auslass abdeckt, wenn das Stellglied in der Ausgangsposition ist.

2. Spender nach Anspruch 1, wobei der Auslass (932) eine flexible Dichtung (842) umfasst.

3. Spender nach Anspruch 2, wobei der Auslöser (824) vorgespannt ist, um die flexible Dichtung (842) zu kontaktieren und mit Druck zu beaufschlagen, wenn das Stellglied in der Ausgangsposition ist.

4. Spender nach Anspruch 1, wobei das Stellglied einen gleitend im Gehäuse (822) angeordneten Schieberkopf (936) und einen an den Auslöser (824) und den Schieberkopf (936) gekoppelten flexiblen Arm (894) aufweist, wobei der Schieberkopf (936) ein Eingriffselement zum Ineingriffnehmen eines ersten Teststreifens (33) im Stapel hat, um den ersten Teststreifen mindestens teilweise durch den Auslass zu bewegen.

5. Spender nach Anspruch 1, ferner mit einer Führungsanordnung (893), die das Stellglied gegen den obersten Teststreifen (33) des Stapels vorspannt, wenn das Stellglied zwischen seiner Ausgangs- und Ausgabeposition ist.

6. Spender nach Anspruch 5, wobei das Stellglied einen Schieberkopf (936) mit mehreren Fingern (937) umfasst.

7. Spender nach einem der vorhergehenden Ansprüche, wobei das Gehäuse (822) eine innere Kassette (884) aufnimmt, in der ein Stapel (834) Teststreifen untergebracht ist.

8. Spender nach einem der Ansprüche 4 bis 7, wobei der Auslöser (824) schwenkbar am Gehäuse (822) montiert ist und an den flexiblen Arm (894) gekoppelt ist.

9. Spender nach einem der Ansprüche 4 bis 8, wobei der flexible Arm (894) einen starren Abschnitt (895) umfasst.

10. Spender nach einem der Ansprüche 7 bis 9, mit einem Paar Führungsschlitzen (938, 939) in der Kassette (884), die entsprechende Führungsstößel (940, 891) am Schieberkopf (936) bzw. den starren Abschnitt (895) des flexiblen Arms (894) aufnehmen.

11. Spender nach einem der Ansprüche 4 bis 10, wobei ein Schubstück-Drucksteuerstößel (950) des Schieberkopfs (936) in einer Raste (945) einer Drucksteuer-Nockenschiene (946) des Gehäuses (822) aufgenommen ist.

12. Spender nach Anspruch 11, wobei die Nockenschiene (946) eine Vorspannfläche bereitstellt, an der der Stößel (950) entlanggleitet, um während des Ausgabehubs des Schieberkopfs (936) nach unten gehenden Druck auf diesen auszuüben.

13. Spender nach Anspruch 12, ferner mit Aussparungen (945, 947) in der Vorspannfläche, die den nach unten gehenden Druck reduzieren, wenn der Schieberkopf (936) in seiner Ausgangs- und seiner Ausgabeposition ist.

14. Spender nach einem der vorhergehenden Ansprüche, wobei der Auslöser (824) durch ein Paar elastische Bänder (960) in die Ausgangs- oder geschlossene Position vorgespannt ist.

**15.** Spender nach einem der Ansprüche 7 - 14, wobei die Kassette (884) und das obere Gehäuse (822) zusammenwirken, um eine im Wesentlichen luftdichte und/oder feuchtigkeitsdichte Einfassung um den Teststreifenstapel (834) zu bilden.

**Revendications**

**1.** Distributeur de bandelettes réactives (800), comprenant :

un logement (822) contenant une pile de bandelettes réactives (834) et définissant une sortie à travers laquelle les bandelettes réactives sont distribuées ;
un actionneur pour déplacer une première bandelette réactive (33) dans la pile au moins partiellement dans la sortie lorsque l'actionneur est déplacé d'une position de repos à une position de distribution ; et
une gâchette (824) pour déplacer l'actionneur de la position de repos à la position de distribution ;
dans lequel la gâchette (824) a une première partie adaptée à être manuellement activée par un utilisateur et **caractérisée en ce que** la gâchette (824) a une deuxième partie (825) qui recouvre la sortie lorsque l'actionneur est dans la position de repos.

**2.** Distributeur selon la revendication 1 dans lequel la sortie (932) comprend un joint d'étanchéité flexible (842).

**3.** Distributeur selon la revendication 2 dans lequel la gâchette (824) est poussée pour venir en contact avec et appliquer une pression sur le joint d'étanchéité flexible (842) lorsque l'actionneur est dans la position de repos.

**4.** Distributeur selon la revendication 1 dans lequel l'actionneur inclut une tête de poussoir (936) disposée de façon coulissante dans le logement (822) et un bras flexible (894) couplé à la gâchette (824) et à la tête de poussoir (936), la tête de poussoir (936) ayant un élément d'engagement pour engager une première bandelette réactive (33) dans la pile pour déplacer la première bandelette réactive au moins partiellement dans la sortie.

**5.** Distributeur selon la revendication 1 comprenant en outre un ensemble de guidage (893) qui pousse l'actionneur contre la bandelette réactive (33) du haut de la pile lorsque l'actionneur est entre ses positions de repos et de distribution.

**6.** Distributeur selon la revendication 5 dans lequel l'actionneur comprend une tête de poussoir (936) ayant une pluralité de doigts (937).

**7.** Distributeur selon l'une quelconque des revendications précédentes, dans lequel le logement (822) reçoit une cassette intérieure (884) qui contient une pile (834) de bandelettes réactives.

**8.** Distributeur selon l'une quelconque des revendications 4 à 7, dans lequel la gâchette (824) est montée de façon pivotante sur le logement (822) et est couplée au bras flexible (894).

**9.** Distributeur selon l'une quelconque des revendications 4 à 8, dans lequel le bras flexible (894) comprend une partie rigide (895).

**10.** Distributeur selon l'une quelconque des revendications 7 à 9, comprenant une paire de fentes de guidage (938, 939) dans la cassette (884) qui reçoivent des suiveurs de guidage (940, 891) correspondants sur la tête de poussoir (936) et la partie rigide (895) du bras flexible (894), respectivement.

**11.** Distributeur selon l'une quelconque des revendications 4 à 10, dans lequel un suiveur de commande de pression de tampon de poussoir (950) de la tête de poussoir (936) est reçu dans un cran (945) d'un rail de came de commande de pression (946) du logement (822).

**12.** Distributeur selon la revendication 11, dans lequel le rail de came (946) est doté d'une surface de poussée le long de laquelle le suiveur (950) coulisse pour appliquer une pression vers le bas sur la tête de poussoir (936) pendant sa course de distribution.

**13.** Distributeur selon la revendication 12, comprenant en outre des renfoncements (945, 947) dans la surface de poussée qui réduisent la pression vers le bas lorsque la tête de poussoir (936) est dans ses positions de repos et de distribution.

**14.** Distributeur selon l'une quelconque des revendications précédentes, dans lequel la gâchette (824) est poussée de façon à être dans la position de repos ou la position fermée par une paire de bandes élastiques (960).

**15.** Distributeur selon l'une quelconque des revendications 7 à 14, dans lequel la cassette (884) et le logement supérieur (822) coopèrent pour former une enceinte substantiellement étanche à l'air et/ou à l'humidité autour de la pile (834) de bandelettes réactives.

FIG. 1

FIG. 2

PRIOR ART

EP 2 005 158 B1

FIG. 2a

PRIOR ART

FIG. 1a

FIG. 3

PRIOR ART

FIG. 4

PRIOR ART

128

126

146

140

142

136

112

FIG. 5a

PRIOR ART

PRIOR ART

FIG. 5b

EP 2 005 158 B1

PRIOR ART       FIG. 6

FIG. 7a

PRIOR ART

EP 2 005 158 B1

FIG. 7b

PRIOR ART

FIG. 7c

PRIOR ART

EP 2 005 158 B1

FIG. 7d PRIOR ART

EP 2 005 158 B1

FIG. 8a

PRIOR ART

80

78

72

74

FIG. 8b

PRIOR ART

FIG. 8c

PRIOR ART

FIG. 9a

PRIOR ART

FIG. 9b

FIG. 10a

PRIOR ART

FIG. 10b

29

FIG. 11a

PRIOR ART

FIG. 11b

FIG. 12a

PRIOR ART

FIG. 12b

14

22    532

530

536

14

FIG.  13

26

38

PRIOR ART

22    532

530

534

FIG.  14

540    536

540

542

534

26

22

648

640

644

646

640

648

630

642

**FIG. 15a**

26

**PRIOR ART**

·22

630

640

642

648

640

**FIG. 15b**

26

**FIG. 15c**

PRIOR ART

EP 2 005 158 B1

FIG. 16

PRIOR ART

FIG. 17a

PRIOR ART

FIG. 17b

PRIOR ART

708

35

20

22

24

30 702

700

FIG. 17c

PRIOR ART

710

**Fig. 18**

Fig. 19

**Fig. 20**

**Fig. 21**

**Fig. 22**

**Fig. 23**

892

895

940

884

950

939

938

937

**Fig. 24**

Fig. 25

**Fig. 26**

**EP 2 005 158 B1**

REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

**Patent documents cited in the description**

- US 2002104849 A1
- WO 2006009534 A1
- US 2005281706 A1